# EUROPEAN PATENT SPECIFICATION

(11) **EP 1 997 558 B1**
(45) Date of publication and mention of the grant of the patent: **23.09.2009**
(21) Application number: 08159282.6
(22) Date of filing: 12.05.2000
(51) Int. Cl.: B01L 3/02, G01N 35/10, A61B 10/00, C12M 1/30, B01L 3/14, B65D 47/00

(54) **Collection device containing specimen-retrieval device**
Sammelvorrichtung mit Probenentnahmevorrichtung
Dispositif de collecte contenant un dispositif de prélèvement de spécimen

(30) Priority: 14.05.1999 US 134265 P
(43) Date of publication of application: 03.12.2008
(62) Divisional of application: 04024555.7
(73) Proprietor: GEN-PROBE INCORPORATED, San Diego, CA 92121-4362 (US)
(72) Inventor: Anderson, Bruce W., Rancho Cucamonga, CA 91730 (US); Iheme, Mordi I., San Diego, CA 92196-0965 (US); Carter, Nick M., Elk Grove, CA 95624-2240 (US); Kacian, Daniel L., San Diego, CA 92124 (US); Clymer, Janice J., Carlsbad, CA 92009 (US); Light, James P., San Diego, CA 92131 (US); Tseo, Gus, San Diego, CA 92130 (US)
(74) Representative: Viering, Jentschura & Partner

(56) References cited:
- US-A- 3 918 435
- US-A- 3 992 150
- US-A- 4 353 868
- US-A- 4 409 988
- US-A- 5 297 599
- US-A- 5 874 048

## Description

The present invention relates to a sealed collection device according to the precharacterizing part of claim 1.

In particular, these sealed collection devices comprise fluid-holding vessels, such as those designed to receive and retain biological specimens for clinical analysis and patient monitoring or diagnosis. A cap is penetrable by a fluid transfer device used to transfer fluids to or from a fluid-holding vessel, where the vessel and cap remain physically and sealably associated during a fluid transfer.

Fluid transfer devices can be used to penetrate the caps. In particular, these fluid transfer devices are adapted to include one or more rib structures which improve the penetration or strength characteristics of the fluid transfer devices and/or which may aid in creating air gaps for venting displaced air from within a collection device. The air gaps may also aid in equilibrating the interior air pressure of a collection device with the ambient air pressure surrounding the collection device.

Collection devices are a type of cap and vessel combination commonly used for receiving and storing biological specimens for delivery to, for example, a clinical laboratory, where the specimens may be analyzed to determine the existence or state of a particular condition or the presence of a particular infectious agent. Types of biological specimens commonly collected and delivered to clinical laboratories for analysis include blood, urine, sputum, saliva, pus, mucous and cerebrospinal fluid. Since these specimen- types may contain pathogenic organisms, it is important to ensure that the collection device is constructed to be essentially leak-proof during transport from the site of collection to the site of analysis. This feature of a collection device is particularly critical when the clinical laboratory and the collection facility are located at sites remote from one another.

To prevent leakage, collection device caps are typically designed to be screwed, snapped or otherwise frictionally fitted onto the vessel component, thereby forming an essentially leak-proof seal between the cap and vessel. In addition to preventing leakage of the specimen, an essentially leak-proof seal formed between the cap and vessel of a collection device will also ameliorate exposure of the specimen to potentially contaminating influences from the surrounding environment. This aspect of a leak-proof seal is important for preventing the introduction of contaminants that could alter the qualitative or quantitative results of an assay.

While a leak-proof seal should prevent specimen seepage during transport, the physical removal of the cap from the vessel prior to specimen analysis presents another opportunity for contamination. When removing the cap, specimen which may have collected on the under-side of the cap during transport could come into contact with a practitioner, possibly exposing the practitioner to harmful pathogens present in the fluid sample. And if the specimen is proteinaceous or mucoid, or if the transport medium contains detergents or surfactants, then a film or bubbles which may have formed around the vessel opening during transport can burst when the cap is removed from the vessel, thereby disseminating specimen into the environment. It is also possible that specimen residue from one collection device, which may be present on the gloved hand of a practitioner, will come into contact with the specimen of another collection device through routine or careless removal of the caps. Another risk is the potential for creating a contaminating aerosol when the cap and vessel are physically separated from one another, possibly leading to false positives or exaggerated results in other specimens being simultaneously or subsequently assayed in the same general area through cross-contamination.

Concerns with cross-contamination are especially acute when the assay being performed involves nucleic acid detection and includes an amplification procedure, such as the well known polymerase chain reaction or transcription-mediated amplification procedure. Since amplification is intended to enhance assay sensitivity by increasing the quantity of targeted nucleic acid sequences present in a specimen, transferring even a minute amount of pathogen-bearing specimen from another container, or target nucleic acid from a positive control sample, to an otherwise negative specimen could lead to a false-positive result.

To minimize the potential for creating contaminating specimen aerosols, and to limit direct contact between specimens and humans or the environment, it is desirable to have a collection device cap which can be penetrated by a fluid transfer device (e.g., pipette tip) while the cap remains physically and sealably associated with the vessel. And, to prevent damage to the fluid transfer device which could effect its ability to predictably and reliably dispense or draw fluids, the cap design should limit the forces necessary for the fluid transfer device to penetrate the cap. Ideally, the collection device could be used in both manual and automated formats and would be suited for use with pipette tips made of a plastic.

Moreover, since the volume of space taken up by the fluid transfer device entering the collection device must displace an equivalent volume of air from within the collection device, means for controlling the rate at which air is released from the collection device as the fluid transfer device penetrates an associated cap would be desirable. In the absence of such means, a pressurized movement of air from the collection device into the surrounding environment could promote the formation and release of potentially harmful or contaminating aerosols, or bubbles when proteins or surfactants are present in the fluid sample. Therefore, a fluid transfer device which facilitates a controlled release of air from a penetrated collection device is needed to prevent or minimize the release of fluid sample in the form of aerosols or bubbles.

U.S. Patent No. 3, 992,150 describes a sealed collection device for test analysis of a substance. The collection device is a cuvet with a foil seal which can be penetrated through an opening through which a charged capillary tube containing the specimen is inserted into the interior of the cuvet. The specimen mixes with the reagent stored in the cuvet. By shaking, the mixing is accelerated, and the empty capillary tube is moved to a vertical inner wall of the cuvet, so that it gets out of the way for photometric testing of the mixed substance in the cuvet.

U.S. Patent No. 3,918,435 discloses an apparatus that includes a removable cap having a swab member. The cap includes a closeable opening formed therein for introducing a liquid into a liquid storage area of the cap. Changing the position of a moveable member of the cap permits the liquid in the liquid storage area to be transferred through a hollow portion of the swab member to a specimen-bearing swab material.

It is an object of the invention to provide a sealed collection device that permits material to be withdrawn from a closed vessel holding a specimen retrieval device,

This object is achieved by the features of claim 1. Further embodiments are claimed in dependent claims 2-15,

Further is provided an integrally molded cap which includes an annular flange adapted to grip an inner or outer side wall surface of a vessel at an open end of the vessel, an annular top wall which is substantially perpendicular to the annular flange, an aperture defined by the inner circumference of the annular top wall, and a conical inner wall which tapers inwardly from the aperture to an apex located substantially at the axis of symmetry of the annular top wall. The annular flange and the conical inner wall each have substantially parallel inner and outer surfaces, and the annular top wall has substantially parallel upper and lower surfaces. In an alternative example, the cap does not include an annular flange adapted to grip a surface of the vessel, but instead the annular top wall forms an annular ring having a lower surface which can be affixed to an upper surface of an annular rim of the vessel by such means as a fixing agent (*e.g*., adhesive) or, alternatively, can be integrally molded with the upper surface of the vessel.

Another set of problems addressed above is solved by providing a fluid transfer device not being part of the invention and having a hollow tubular body which includes one or more rib structures on an outer surface, inner surface, or both inner and outer surfaces of the fluid transfer device. When the rib structures are located on the outer surface, they are expected to create ventilation channels between the outer surface of the fluid transfer device and the penetrated surface material of the cap. These ventilation channels were found to advantageously facilitate the release or air from within the penetrated collection device of the invention, while minimizing the formation and/or release of fluid sample in the form of an aerosol or bubbles. Rib structures on the outer surface of a fluid transfer device are also expected to improve the strength characteristics of the fluid transfer device and to reduce the force required to pierce a penetrable cap. These strength and force reduction characteristics are also expected to be associated with fluid transfer devices having rib structures positioned on an inner surface.

In a second embodiment of the present invention, the conical inner wall of the cap has a single angle with respect to the axis of symmetry of the annular top wall. The cap of this embodiment is penetrable by a fluid transfer device consisting of a plastic pipette tip and does not significantly impair the pipette tip's ability to accurately draw a fluid substance after the cap has been penetrated by the pipette tip.

In a third embodiment of the present invention, the conical inner wall of the cap is adapted to include a plurality of striations which extend radially outwardly from the apex, or from one or more start-points near the apex, of the conical inner wall. Each of the striations extends partially or fully from the apex, or from a start-point near the apex, of the conical inner wall to an outer circumference of the conical inner wall. The striations may be in the form of channels, grooves, etchings or series of perforations on at least one surface of the conical inner wall, and the thickness of each striation is less than the thickness of non-striated portions of the conical inner wall. The striations were advantageously found to reduce the force needed to penetrate the cap and to concomitantly create air passageways between portions of the conical inner wall and the fluid transfer device as sections of conical inner wall defined by the striations pealed away from the fluid transfer device upon penetration.

In a fourth embodiment of the present invention, the annular flange of the cap has an upper portion which extends vertically above the annular top wall, so that the upper surface of the annular top wall can serve as a ledge for positioning and maintaining a wick material substantially above the conical inner wall and within the annular flange. The wick material may be of any material or combination of materials included to limit the spread of bubbles, aerosols and/or to provide a wiping feature for removing fluid present on the outside of a fluid transfer device as it is being withdrawn through the cap of a collection device. The wick material preferably draws fluid away from the fluid transfer device by means of capillary action.

In a fifth embodiment of the present invention, the cap further includes a seal which is affixed to the annular top wall or an annular top surface of the upper portion of the annular flange, or is otherwise fixedly positioned within an inner surface of the annular flange (*e*.*g*., a hollow-centered resin disk with a seal affixed thereto and sized to frictionally fit within an inner surface of the annular flange and to permit passage therethrough by a fluid transfer device). While the seal is penetrable with a fluid transfer device, the seal may be applied to or associated with the cap in such a way that it can be separated from the cap prior to penetration with a fluid transfer device. The seal may be provided to keep contaminants off of the conical inner wall (and, if present, the wick material), to aid in preventing aerosols from being released from the collection device when the cap is penetrated by a fluid transfer device and/or to retain the wick material within the annular flange. As indicated, the seal is preferably made of a penetrable material, such as a metallic foil or plastic, and completely or partially covers the conical aperture prior to penetration.

In an example not being part of the invention, a cap is provided which can be penetrated by a plastic pipette tip with the application of a force of less than about 8 pounds to a surface of the cap. This particular cap also includes a wick material positioned above or below a penetrable surface material of the cap and which requires less than about 4 pounds pressure for the pipette tip to pass through. The wick material is arranged in the cap so that it can at least partially trap aerosols or bubbles escaping from an associated vessel during and/or after penetration of the cap by the plastic pipette tip.

In another example not being part of the present invention, an overcap containing a wick material is provided which can be positioned over a cap used in connection with the present invention. An annular top wall of the overcap includes an inner circumference which defines an aperture which has been sized to receive a fluid transfer device for penetrating the conical inner wall of the cap. Rib structures may be further included on an inner surface of an annular flange of the overcap to provide a frictional fit between the inner surface of the overcap and the annular outer flange of the cap. A seal may also be applied to the annular top wall of the overcap to further minimize aerosol or bubble release from a collection device once the cap has been penetrated and/or to retain the wick material within the annular flange of the overcap. The overcap, which provides the benefits of aerosol and bubble containment in a separate component, may be optionally employed, for example, with a collection device having a cap lacking a wick material when the sample to be removed and analyzed is suspected of containing a target nucleic acid analyte which is to be amplified before a detection step is performed.

In another example not being part of the present invention, a fluid transfer device is provided which may be used to facilitate penetration of the caps and overcap used in connection with the present invention and/or which may improve venting of air as it is being displaced from a vessel by an entering fluid transfer device. This particular fluid transfer device is hollow in construction (although the fluid transfer device may be outfitted with an aerosol impeding filter), designed to be engaged by a probe or extension associated with a robotic or manually operated fluid transfer apparatus for drawing and/or dispensing fluids, and includes one or more rib structures. These rib structures extend outward from an outer surface of the body of the fluid transfer device and, preferably, in a generally vertical direction from a point or points at or near the distal end of the fluid transfer device. The increased strength and mass attributable to these rib structures may reduce the force required to puncture a penetrable cap and, in some cases, will permit the fluid transfer device to perform acceptably in multiple penetrations.

In another example not being part of the present invention, a plastic pipette tip is provided which has hollow tube and cone sections for the passage of air and/or fluids therethrough and one or more lower rib structures located on the cone section which extend outward from an outer surface of the cone section. These lower rib structures are expected to provide the same benefits attributable to the seventh embodiment of the present invention.

In another example not being part of the present invention, a plastic pipette tip is provided which has hollow tube and cone sections for the passage of air and/or fluids therethrough and one or more lower rib structures located on the cone section which extend inward from an inner surface of the cone section. These lower rib structures are expected to facilitate penetration of the caps and overcap used in connection with the present invention.

In another example not being part of the present invention, a plastic pipette tip is provided which has hollow tube and cone sections for the passage of air and/or fluids therethrough and one or more upper rib structures on the tube section which extend outward from an outer surface of the tube section, with at least one of these upper rib structures having a terminus at or near the distal end of the tube section. These upper rib structures are designed to aid in the formation of air gaps between the penetrated surface material of a cap and the pipette tip to facilitate the movement of air displaced from the interior of an associated collection device as it is being entered by the pipette tip and/or so that the air pressures inside and outside of the collection device can equilibrate upon penetration of the cap.

In another example not being part of the present invention, a plastic pipette tip is provided which combines the lower rib and upper structures of the eighth and tenth or ninth and tenth embodiments described above, where the lower rib structures may be distinct from the upper rib structures or pairs of lower and upper rib structures may form continuous rib structures extending from a point or points on the cone section to a point or points on the tube section.

In a sixth embodiment of the present invention, a method according to claim 12 is provided for obtaining a fluid substance from the sealed collection device according to any of claims 1 to 10. Once the cap has been penetrated, substance present in a vessel of the collection device is withdrawn by the fluid transfer device before being removed from the collection device.

Further embodiments of the method are set out in dependent claims 13-15.

Caps may be used in connection with the present invention may be provided in packaged combination with at least one of a vessel, a reagent (*e*.*g*., transport medium or positive control), an overcap, a fluid transfer device and a specimen retrieval device.

These and other features, aspects, and advantages of the present invention will become apparent to those skilled in the art after considering the following detailed description, appended claims and accompanying drawings.

### BRIEF DESCRIPTION OF THE DRAWINGS

FIG. 1 shows an exploded perspective view of a collection device 10, including a preferred cap 20A and optional seal 80 of the present invention, in which a vessel 50 has a thread 54 which is mated with a thread 42 on the cap 20A (not visible in this figure).
FIG. 2 shows an enlarged top plan view of the cap 20A depicted in FlG. 1, in which a conical inner wall 33 of the cap 20A includes partially extending striations 35.
FIG. 3 shows an enlarged bottom plan view of the cap 20A depicted in FIG. 1, in which the conical inner wall 33 of the cap 20A includes partially extending striations 35.
FIG. 4 shows an enlarged top plan view of another cap 20B of the present invention, in which the conical inner wall 33 of the cap 20B includes fully extending striations 35.
FIG. 5 shows an enlarged partial section side view of the cap 20A and vessel 50 depicted in Figs. 1 and 2, taken along the 5-5 line thereof, with an optional seal 80 applied to an annular top wall 22 of the cap 20A.
FIG. 6 shows an enlarged partial section side view of a cap 20C and vessel 50, where the cap 20C includes an extended annular outer flange 40A and an optional seal 80 applied to an annular top surface 48, a wick 90 contained within an upper portion 46 of the annular outer flange 40A, and the vessel 50 and cap 20C include interlocking rims 55, 43.
FIG. 7 shows the enlarged partial section side view of the cap 20A and vessel 50 depicted in FIG. 5 with a fluid transfer device 70 (*i*.*e*., pipette tip with a beveled tip 71) passing therethrough and a swab 130 positioned along an inner surface 59 of a side wall 58.
FIG. 8 shows an enlarged top plan view of the cap 20A depicted in FIG. 5 after the fluid transfer device 70 shown in FIG. 7 has been removed therefrom.
FIG. 9 shows an enlarged partial section side view of an overcap 100 in combination with the cap 20A and vessel 50 shown in FIG. 5, where the overcap 100 contains a wick 90 located beneath a lower surface 105 of an annular top wall 104 and an optional seal 80 applied to an upper surface 106 of the annular top wall 104.
FIG. 10 shows an enlarged side elevation view of a pipette tip 70A not being part of the present invention having lower rib structures 151 A, 152A and a beveled tip 71A.
FIG. 11 shows another enlarged side elevation view of the pipette tip depicted in FIG. 10, including two of the lower rib structures 152A and a fluid receiving orifice 161.
FIG. 12 shows an enlarged perspective view of a distal portion of a cone section 166 of the pipette tip 70A depicted in FIG. 10.
FIG. 13 shows an enlarged bottom section view of the pipette tip 70A depicted in FIG. 11, taken along the 13-13 line thereof.
FIG. 14 shows an enlarged side elevation view of a pipette tip 70B not being part of the present invention having lower rib structures 151B, 152B with tapered or blunt-ended distal termini 162B, 163B and upper rib structures 174.
FIG. 15 shows another enlarged side elevation view of the pipette tip 70B depicted in FIG. 14, including two of the lower rib structures 152B, two of the upper rib structures 174, and a fluid receiving orifice 161.
FIG. 16 shows an enlarged perspective view of a distal portion of the pipette tip 70B depicted in FIG. 14.
FIG. 17 shows an enlarged side elevation view of a pipette tip 70C not being part of the present invention having upper rib structures 174 and lower rib structures 151C, 152C on an inner surface 157 of a cone section 166 of the pipette tip 70C.
FIG. 18 shows an enlarged side section view of the pipette tip 70C of FIG. 17, taken along the 17-17 line thereof, including the lower rib structures 151C, 152C positioned on the inner surface 157 of the cone section 166.
FIG. 19 shows an enlarged bottom section view of the pipette tip 70C depicted in FIG. 17, taken along the 19-19 line thereof.
FIG. 20 shows an enlarged side elevation view of a pipette tip 70D not being part of the present invention having continuous rib structures 176 extending from the distal end of a cone section 166 to the proximal end of a tube section 167.
FIG. 21 shows an enlarged side elevation view of a pipette tip 70E not being part of the present invention having upper rib structures 174 on a tube section 167 and no lower rib structures on a cone section 166.

### DETAILED DESCRIPTION OF THE INVENTION

With reference to the figures, the cap 20A-C being part of the present invention can be combined with a vessel 50 to receive and store fluid specimens for subsequent analysis, including analysis with nucleic acid-based assays or immunoassays diagnostic for a particular pathogenic organism. When the desired specimen is a biological fluid, the specimen can be, for example, blood, urine, saliva, sputum, mucous or other bodily secretion, pus, amniotic fluid, cerebrospinal fluid or seminal fluid. However, the present invention also contemplates materials other than these specific biological fluids, including, but not limited to, environmental samples (*e.g.*, water), chemicals and assay reagents, as well as solid substances which can be dissolved in whole or in part in a fluid milieu (e.g., tissue specimens, powders, particles, granules and food products). Vessels 50 used with the cap 20A-C are capable of forming a substantially leak-proof seal with the cap 20A-C and can be of any shape or composition, provided the vessel 50 is shaped to receive and retain the material of interest (*e*.*g*., fluid specimen or assay reagents). Where the vessel 50 contains a specimen to be assayed, it is especially important that the composition of the vessel 50 be essentially inert so that it does not significantly interfere with the performance or results of an assay.

The cap 20A-C being part of the present invention may be prepared from a number of different polymer and heteropolymer resins, including, but not limited to, polyolefins (e.g., high density polyethylene ("HDPE"), low density polyethylene ("LDPE"), a mixture of HDPE and LDPE, or polypropylene), polystyrene, high impact polystyrene and polycarbonate. An example of an HDPE is sold under the tradename Alathon M5370 and is available from Polymerland of Huntsville, North Carolina; an example of an LDPE is sold under the tradename 722 and is available from The Dow Chemical Company of Midland, Michigan; and an example of a polypropylene is sold under the tradename Rexene 13T10ACS279 and is available from the Huntsman Corporation of Salt Lake City, Utah. Although LDPE is a softer, more malleable material than HDPE, the softness of LDPE creates more frictional resistance when a threaded cap is screwed onto a threaded vessel than when a cap is formed of the more rigid HDPE material. And, while a cap made of HDPE is more rigid than one made of LDPE, it is this rigidity that makes an HDPE cap more difficult to penetrate than one made of LDPE. Although the cap 20A-C being part of the present invention is preferably comprised of HDPE, it can also be comprised of a combination of resins, including, for example, a mixture of LDPE and HDPE, preferably in the range of about 20% LDPE:80% HDPE to about 50% LDPE:50% HDPE by volume.

Based on the guidance provided herein, those skilled in the art will be able to select a resin or mixture of resins providing the greatest combination of rigidity and penetrability characteristics under specific conditions of use, without requiring anything more than routine experimentation. Additionally, skilled artisans will realize that the range of acceptable cap 20A-C resins will also depend on the nature of the resin used to form the collection device 10, since the properties of the resins used to form these two components will affect how well the cap 20A-C and vessel 50 of the collection device 10 can form a leak proof seal and the ease with which the cap 20A-C can be securely screwed onto the vessel 50. (The currently preferred vessel 10 is made of polypropylene.) And to further fine-tune the rigidity and penetrability characteristics of a cap 20A-C, those skilled in the art will recognize that the molded material may be treated by, for example, heating, irradiating or quenching.

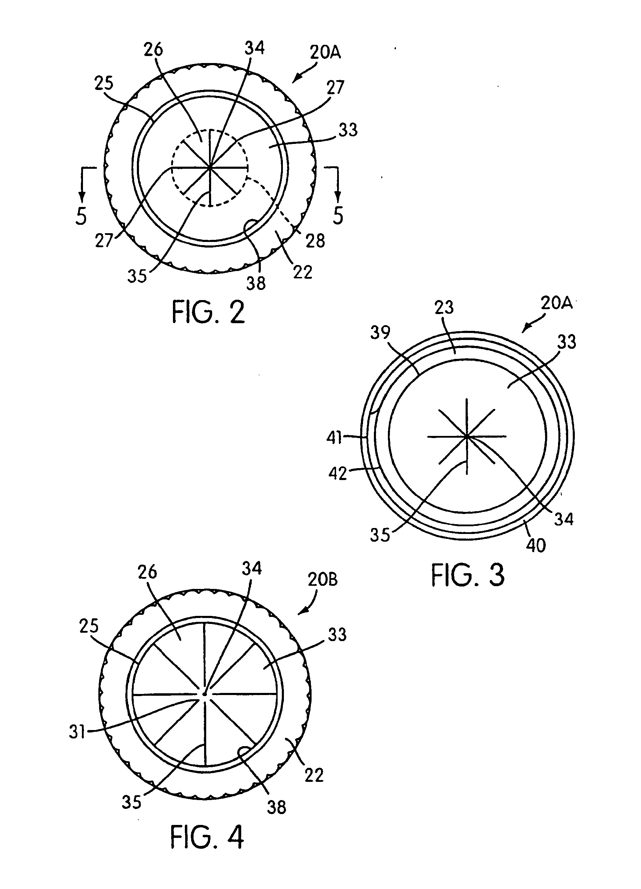

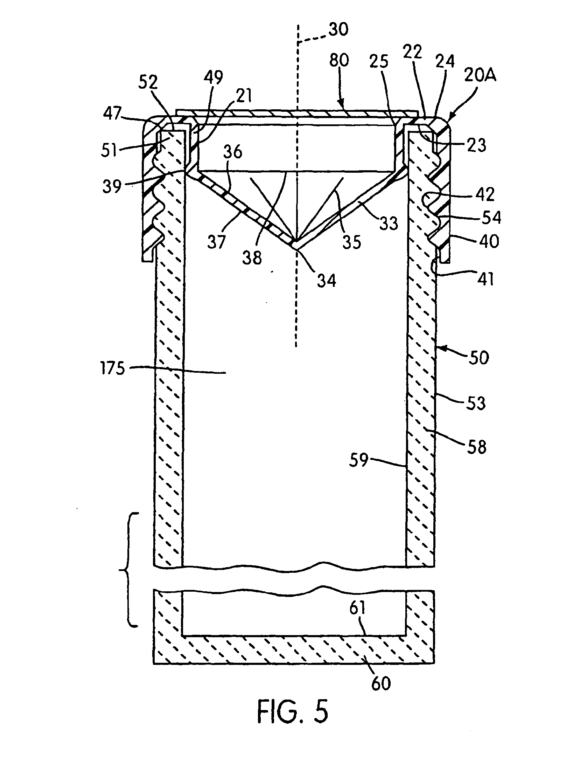

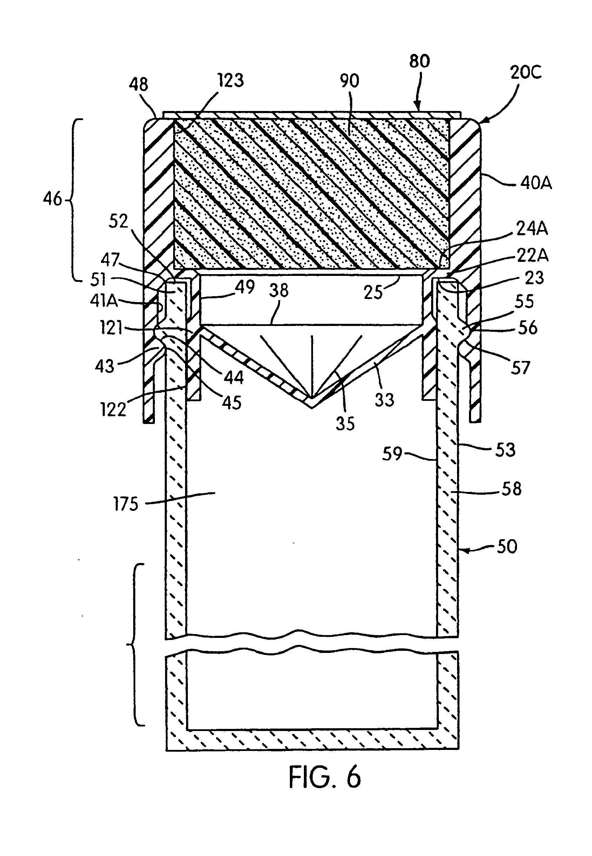

Regardless of the type or mixture of resins chosen, the cap 20A-C is preferably injection molded as a unitary piece using procedures well-known to those skilled in the art of injection molding, including a multi-gate process for facilitating uniform resin flow into the cap cavity used to form the shape of the cap 20A-C. Uniform resin flow is desirable for achieving consistency in thickness, which is especially important for the penetrable surface of the cap 20A-C. After preparing the integrally molded cap 20A-C, a wick 90 may be provided within the aperture defined either by an inner circumference 25 of the annular top wall 22 *(see* FIG. 2) or by the circumference of an inner surface 123 of the upper portion 46 of the annular outer flange 40A (*see* FIG. 6). The wick 90 is preferably positioned above the conical inner wall 33 of the cap 20A-C to aid in further containing and limiting the dissemination of aerosols outside of the collection device 10. In addition, a seal 80 may be applied to an upper surface 24 of an annular top wall 22 (cap 20A-B) or an annular top surface 48 (cap 20C) to provide a protective cover over the aperture above the conical inner wall 33 of the cap 20A-C (and to retain the wick 90, if present, in the cap 20A-C), as depicted in Figs. 5 and 6.

While the outer circumference 38 of the conical inner wall 33 may coincide with the inner circumference 25 of the annular top wall 22 in a single plane (not shown), such that there is no annular inner flange 49, the cap 20A of FIG. 5 is used in a preferred embodiment since it includes an annular inner flange 49 which extends substantially vertically from the outer circumference 38 of the conical inner wall 33 to the inner circumference 25 of the annular top wall 22, providing the additional vertical space in the aperture required for receiving a wick 90. However, when a wick 90 is to be included in the cap 20A-C, an extension of the annular outer flange 40A, as illustrated in FIG. 6, is particularly preferred. In this arrangement, the annular outer flange 40A has an upper portion 46 located above the upper surface 24A of the annular top wall 22A, and is constructed so that an inner surface 123 of the upper portion 46 of the annular outer flange 40A terminates at the upper surface 24A of the annular top wall 22A. With this preferred arrangement, the inner circumference 25 of the annular top wall 22A is smaller than the circumference defined by the inner surface 123 of the upper portion 46 of the annular outer flange 40A. In this way, the upper surface 24A of the annular top wall 22A can function as a ledge for positioning and maintaining a wick 90 above the conical inner wall 33.

Inclusion of a wick 90 not only helps to retard the movement of aerosols from the vessel 50 to the environment, it can also be constructed to perform a wiping action on the outside of the fluid transfer device 70 as the fluid transfer device 70 is being removed from the vessel 50 and cap 20A-C. In a preferred mode, the wick 90 functions to draw fluids away from the outside of the fluid transfer device 70 by means of capillary action. As used herein, however, the term "wick" refers to a material which performs a wiping function to remove fluids present on the outside of a fluid transfer device 70 and/or an absorbing function to hold fluids removed from the outside of a fluid transfer device 70. Examples of wick 90 materials which may be used with the cap 20A-C being part of the present invention include, but are not limited to, pile fabrics, sponges, foams (with or without a surface skin), felts, sliver knits, Goretex®, Spandex®, and other materials, both natural and synthetic. These materials may also be mechanically or chemically treated to further improve the intended functions of the wick 90. For example, napping may be used to increase the surface area and, therefore, the fluid holding capacity of a wick 90. The material of the wick 90 might also be pre-treated with a wetting agent, such as a surfactant, to lower the surface tension of a fluid present on an outer surface of a fluid transfer device 70. An acrylic binder might be used, for example, to actually bind the wetting agent to the wick 90 material.

If the fluid transfer device 70 is not of uniform diameter, as is the case with most standard air displacement pipette tips, then it is preferred that the material of the wick 90 be able to reform around smaller diameter sections of the fluid transfer device 70 as it is being removed from a collection device 10. Thus, materials such as pile fabric, sponges, foams and Spandex are preferred because of their ability to rebound rapidly after exposure to compressive forces. Pile fabric is a particularly preferred wick 90 material, an example of which includes a 3/8 inch (9.53 mm) pile fabric of acrylic construction which is available from Roller Fabrics of Milwaukee, Wisconsin as Part No. ASW112. Other acceptable pile fabrics are made of acrylic and polyester materials, range in size from 1/4 inches (6.35 mm) to 5/16 inches (7.95 mm) and are available from Mount Vernon Mills, Inc. of LeFrance, South Carolina as Part Nos. 0446, 0439 and 0433. The wick 90 material is preferably inert with respect to a fluid sample contained within the vessel 50.

Because wick 90 materials are designed to draw fluids away from the exterior of the fluid transfer device 70 and/or to capture fluids in the form of aerosols and/or bubbles, the material and dimensions of the wick 90 must be chosen to avoid excessive saturation with fluid. If the wick 90 becomes overly saturated, fluid may not be adequately wiped form the exterior of fluid transfer devices 70 and/or bubbles may be produced upon insertion of fluid transfer devices 70 and/or displacement of air from within the collection device 10. Thus, it is important to adapt the size and adsorptive properties of the wick 90 in order to achieve adequate wiping and aerosol and/or bubble containment for a given cap 20A-C configuration, fluid transfer device 70 and fluid substance given the number of anticipated fluid transfers the wick 90 will participate in. Hence, as the volume of liquid expected to be presented to the wick 90 in a given application increases, the amount of wick 90 material and/or its absorbancy may need to be adjusted so that the wick 90 does not become overly saturated during use.

It is also important that the wick 90 be constructed and arranged in the cap 20A-C so that the flow of air out of the collection device 10 is relatively unimpeded. This property is not only important when the wick 90 is dry but also when it has absorbed the maximum volume of fluid expected for a given application. However, it should be recognized that this property of the wick 90 needs to be balanced with the requirement that the wick 90 have sufficient density to trap escaping aerosols and/or bubbles. Therefore, those skilled in the art will need to select or design wick 90 materials having matrices that are capable of trapping aerosols and bubbles, while simultaneously permitting air to be vented from the collection device 10 once the underlying surface material of the penetrable cap 20A-C has been pierced.

As shown in FIG. 6, the wick 90 is preferably sized to fit beneath the horizontal plane of the annular top surface 48 of the cap 20C (or the upper surface 24 of the annular top wall 22 of the cap 20A-B) and above the annular top wall 22A, where it is restrained by the seal 80 and annular top wall 22A. To better ensure that the wick 90 is not substantially moved from this location by frictional contact with a fluid transfer device 70 penetrating or being removed from the cap 20A-C, at least one annular shelf (not shown) above or below the wick 90 and extending inwardly from an inner surface 21, 123 of the cap 20A-C could be provided. Such an annular shelf would be particularly advantageous where the cap 20A-C does not include a seal 80. Moreover, in an effort to further impede the mobility of the wick 90, the wick could be glued or otherwise adhered to at least one of the suggested annular shelves, the seal 80 and the annular top wall 22A. Alternatively, the wick 90 may be glued or otherwise adhered to the inner surface 123 of the upper portion 46 of the annular outer flange 40A.

In a preferred embodiment, the aperture defined by the inner surface 123 of the upper portion 46 of the annular outer flange 40A is sealed with a metallic foil 80 (or foil laminate) using, for example, a pressure sensitive adhesive which is applied to the annular top surface 48 (cap 20C) or the upper surface 24 of the annular top wall 22 (cap 20A-B). The material and configuration of the wick 90 should be such that it creates minimal frictional interference with the fluid transfer device 70 when it is inserted into or withdrawn from the cap 20A-C and vessel 50. In the case of a sponge or foam, for example, this may require boring a hole or creating one or more slits in the center of the wick 90 which are sized to minimize frictional interference but, at the same time, to provide some frictional interference with the fluid transfer device 70 so that aerosol transmission is limited and the wiping action is performed. If a pile fabric is employed as the wick 90, the pile fabric is preferably arranged so that the free ends of individual fibers are oriented inward toward one another and away from the pile fabric backing which is arranged in the cap 20A-C in a generally circular fashion within an inner surface 21 of the annular inner flange 49 or the inner surface 123 of the upper portion 46 of the annular outer flange 40A. Care should be taken not to wind the pile fabric so tightly that it will create excessive frictional interference with a fluid transfer device 70 being moved into or out of the vessel 50 and cap 20A-C, thereby substantially impeding movement of the fluid transfer device. The movement of a fluid transfer device 70 is deemed "substantially impeded" if the force required to penetrate the wick 90 is greater than the force required to penetrate the cap 20A-C which contains it. The force required to penetrate the wick 90 is preferably less than about 4.0 pounds (1.81 kg), more preferably less than about 2.0 pounds (0.91 kg), even more preferably less than about 1.0 pound (0.45 kg), and most preferably less than about 0.5 pounds (0.23 kg).

When the seal 80 is included, it is preferably made of a plastic film (*e*.*g*., biaxial polypropylene) or metallic foil material (*e*.*g*., aluminum foil), which can be affixed to the annular top surface 48 (cap 20C) or the upper surface 24 of the annular top wall 22 (cap 20A-B) using means well known to those skilled in the art, including adhesives. A metallic seal 80 may further include a plastic liner, such as a thin veneer of HDPE applied to one or both surfaces of the metallic material, which promotes attachment of the seal 80 to the annular top wall 22 when a heat induction sealer is used. Heat induction sealing is a well known process and involves the generation of heat and the application of pressure to the surface being sealed, which, in this case, is the annular top surface 48 (cap 20C) or the upper surface 24 of the annular top wall 22 (cap 20A-B). The heat is used to soften the material of the annular top surface 48 or the annular top wall 22 (and the seal 80 if it includes a resin veneer) for permanently receiving the seal 80, and pressure is applied to the cap 20A-C while the seal 80 becomes affixed to the annular top surface 48 or the upper surface 24 of the annular top wall 22. Any known ultrasonic welding procedures using either high frequency or high amplitude sound waves may also be used to affix the seal 80 to the cap 20A-C.

Where aerosol release from the collection device 10 is a particular concern, the seal 80 may be used to further reduce the amount of aerosol which can be released from the collection device 10 when the conical inner wall 33 of the cap 20A-C is penetrated. Under these circumstances, the material selected for the seal 80 should experience minimal tearing when the fluid transfer device 70. such as a pipette tip or fluid-transporting needle, passes through it. Some tearing, however, is desirable to avoid creating a vacuum within the collection device 10 once the cap 20A-C has been penetrated. An example of a pipette that can be used with the cap 20A-C being part of the present invention is a Genesis series 1000 µl Tecan-Tip (with filter), available from Eppendorf-Netherler-Hinz GmbH of Hamburg, Germany. In addition to limiting the amount of aerosol released from the collection device 10, the seal 80 can also serve to protect the conical inner wall 33 of the cap 20A-C and/or the inserted wick 90 from undesirable environmental contaminants.

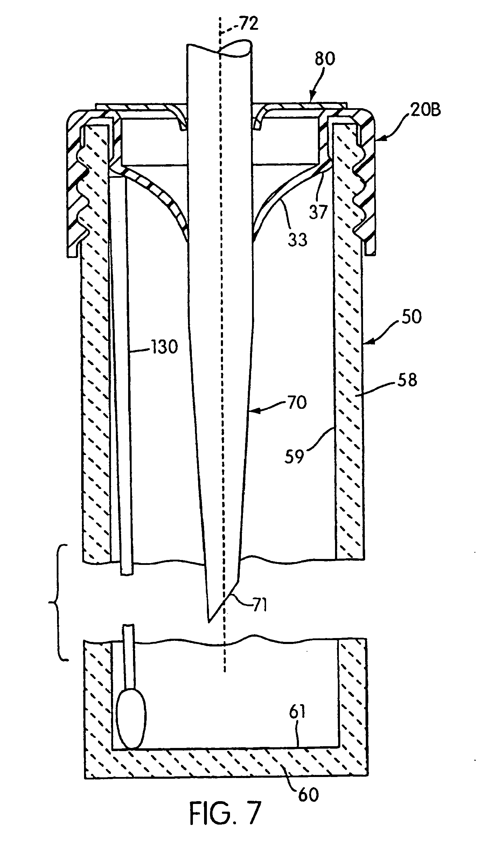

As exemplified in FIG. 5, the cap 20A-C being part of the present invention is designed to include a conical inner wall 33 which tapers inwardly from the aperture which is defined by the inner circumference 25 of the annular top wall 22, (*see* FIG. 2), to an apex 34 located substantially at the axis of symmetry 30 of the annular top wall 22. The shape of the conical inner wall 33 aids in guiding the fluid transfer device 70 to the apex 34 in the conical inner wall 33 where the fluid transfer device 70 will penetrate the cap 20A-C, as shown in FIG. 7. Therefore, the angle of the conical inner wall 33 should be chosen so that penetration of the apex 34 by the tip 71 of the fluid transfer device 70 is not substantially impeded. Thus, the angle of the conical inner wall 33, with respect to the axis of symmetry 30, is preferably about 25° to about 65°, more preferably about 35° to about 55°, and most preferably about 45° ± 5°. Ideally, the conical inner wall 33 has a single angle with respect to the axis of symmetry 30.

As shown in FIG. 7, it was discovered that the shape of the conical inner wall 33 of the cap 20A-C being part of the present invention can also function to position a specimen retrieval device, such as a specimen-bearing swab 130, along an inner surface 59 of a side wall 58 of the vessel 50 so that it does not substantially interfere with the movement of a fluid transfer device either into or out of a collection device 10. Such interference may constitute any physical contact between the swab 130 and the fluid transfer device as it enters or leaves the collection device 10, or it may simply mean that the position of the swab 130 does not prevent the fluid transfer device from entering and drawing an accurate volume of fluid sample from the collection device 10. To ensure that the swab 130 is sufficiently isolated from the pathway of the fluid transfer device within the collection device 50, the swab 130 will need to be sized so that it fits snugly beneath an outer surface 37 of the conical inner wall 33 and along the inner surface 59 of the side wall 58 of the vessel *(see* FIG. 7) when the collection device 10 is fully assembled. One way to achieve this snug fit is to use a swab 130 which has been manufactured to include a mid-section score line (not shown), thereby permitting an upper portion of the swab 130 to be manually snapped-off and discarded after use, leaving only the specimen-bearing, lower portion of the swab 130 in the collection device 10. The precise location of the score line on the swab 130 will need to be determined based upon the interior dimensions of the collection device 10 when the cap 20A-C is frictionally-fitted onto the vessel 50. Breakable swabs are fully described in U.S. Patent No. 5,623,942, the contents of which are hereby incorporated by reference herein.

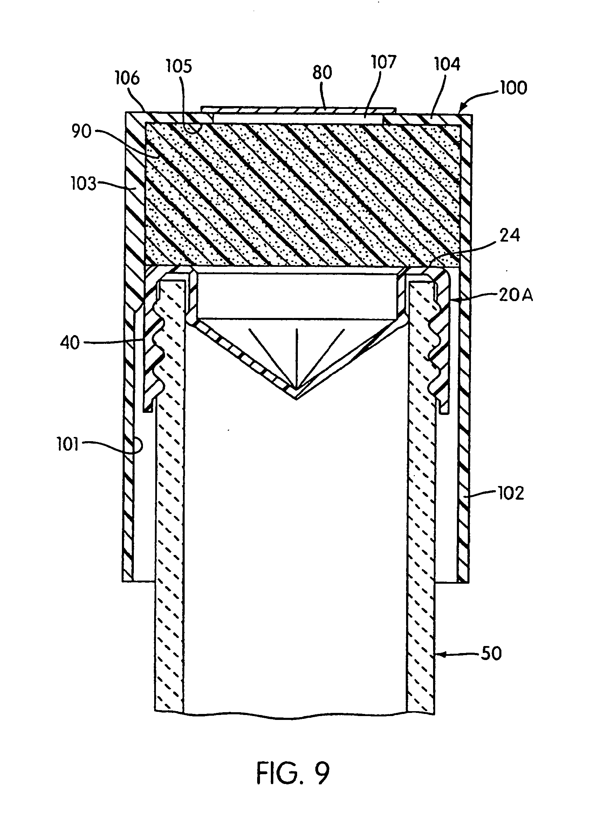

Another example is depicted in FIG. 9 and includes an overcap 100, preferably constructed of an injected molded plastic which has been adapted to fit over the cap 20A-B shown in Figs. 2-5 (generally without the seal 80), preferably forming a frictional fit between the annular outer flange 40 of the cap 20A-B and a portion of an inner surface 101 of the annular flange 102 of the overcap 100. To achieve this frictional fit between the cap 20A-B and the overcap 100, the overcap 100 may be configured to include one or more ribs 103 which extend inwardly from the inner surface 101 af the overcap 100 and which make physical contact with the annular outer flange 40 when the overcap 100 is positioned over the cap 20A-B. The overcap 100 of this example contains a wick 90 which is fixedly positioned within the inner surface 101 of the annular flange 102 and beneath a lower surface 105 of an annular top wall 104 of the overcap 100 by means of, for example, a frictional fit or adhesive. The wick 90 can be used for any of the reasons discussed hereinabove and may be made of any material having the aerosol retarding or wiping properties referred to *supra.* A seal 80 may also be included, for instance, to act as an additional barrier to the flow of aerosols from the collection device 10 when the conical inner wall 33 is penetrated by a fluid transfer device 70. When used, the seal 80 is preferably applied to the annular top wall 104 of the overcap 100 using conventional methods, including the heat induction and ultrasound methods mentioned hereinabove. To permit penetration of the conical inner wall 33 of the cap 20A-B by a fluid transfer device 70, the annular top wall 104 of the overcap 100 includes an aperture 107 sized to receive the fluid transfer device 70, where the size of the aperture 107 is large enough so that the annular top wall 104 does not interfere with the movement of the fluid transfer device 70 into and out of the vessel 50 of the collection device 10.

Included in the conical inner wall 33 of the preferred cap 20A-C are a plurality of striations 35 which extend radially outwardly from the apex 34, or from one or more start-points 31 near the apex *(see, e*.*g*., FIG. 4), toward the outer circumference 38 of the conical inner wall 33. Where a striation 35 extends from a start-point 31 "near" the apex 34, the start-point 31 is located on the conical inner wall 33 within a distance of at least about 0.05 inches (1.27 mm) from the apex 34, and preferably within a distance of at least about 0.025 inches (0.635 mm) from the apex 34. When the start-points 31 of the striations 35 in the conical wall 33 are all positioned slightly away from the apex 34, it was discovered that a more uniform resin thickness in the apex 34 could be achieved during the injection molding process and that the striations 35 tended to "open" more evenly upon penetration, as described *infra.*

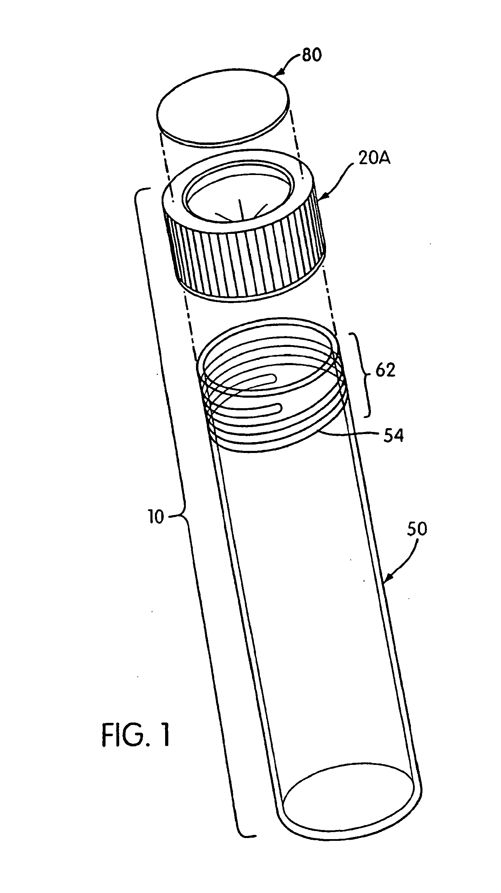

The striations 35, as shown in Figs. 1-6, 8 and 9, were discovered to enhance penetration of the conical inner wall 33 by the fluid transfer device 70. Examples of striations 35 in the conical inner wall 33 of the cap 20A-C include channels, grooves, etchings or a series of perforations which can be formed on a core pin using known injection molding techniques or which can be physically "etched" or pierced with a cutting tool following formation of the cap 20A-C using well known techniques. The striations 35 may be of any number sufficient to improve penetrability of the conical inner wall 33 of the cap 20A-C, as determined by a reduction in the force required to penetrate a cap 20A-C. Notwithstanding, the number of striations 35 on a cap 20A-C is preferably from about 3 to about 12, more preferably from about 6 to about 10, and most preferably about 8. In one embodiment shown in FIG. 2, the striations 35 all extend an approximately equal distance from the apex 34 to form generally wedge-shaped sections 26 on the conical inner wall 33 when an imaginary line 28 is circumferentially drawn to connect the end-points 27 of the striations 35. A similar configuration is shown for the fully extended striations 35 in FIG. 4. These wedge-shaped sections 26 illustrated in Figs. 2 and 4 are preferably of the same approximate size and shape. The striations 35 may be formed on either the inner surface 36 of the conical inner wall 33 or the outer surface 37 of the conical inner wall 33 or both surfaces 36, 37.

When striations 35 are included with a cap 20A-C being part of the present invention, the force of a fluid transfer device 70 needed to penetrate a cap 20A-C having a plurality of striations 35 must be less than the force needed to penetrate a cap of the same material, shape and dimensions but having no striations 35. Preferably, the force required to penetrate a cap 20A-C having a plurality of striations 35 is no more than about 95% of the force required to penetrate a cap of identical material, shape and dimensions but which has no striations 35. To "penetrate" a cap 20A-C, a fluid transfer device 70 need only pierce the conical inner wall 33, preferably at or near the apex 34. The force value is more preferably no more than about 85%, even more preferably no more than about 75%, and most preferably no more than about 65%. This value is ideally no more than about 50% when the fluid transfer device 70 includes a beveled tip 71, as shown if FIG. 7. For all caps being part of the present invention, whether striated or unstriated, the preferred force needed by a plastic fluid transfer device 70 (*i*.*e*., pipette tip) to penetrate the cap is less than about 8.0 pounds (3.63 kg), more preferably less than about 6.0 pounds (2.72 kg), and most preferably less than about 4.0 pounds (1.80 kg). The force needed to penetrate a cap can be determined using the equipment, materials and protocol described in the Example *infra*.

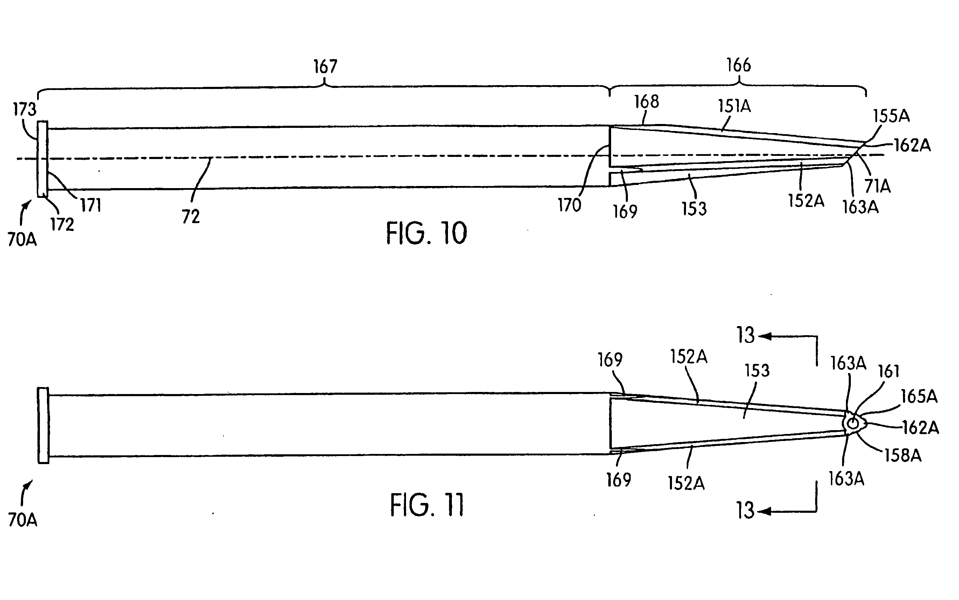

A particularly preferred fluid transfer device for use with the cap 20A-C being part of the present invention is a pipette tip 70A-C shown in Figs. 10-19. This pipette tip 70A-C includes one or more lower rib structures 151A-C, 152A-C which are preferably, although not necessarily, generally vertical in orientation (when used to describe an aspect of a pipette tip 70A-E *infra,* "vertical" shall mean the direction of the axis of symmetry 72 extending from the distal end toward the proximal end of the pipette tip 70A-E, as shown, for example, in FIG. 10) and extend outward from an outer surface 153 at the distal end of the pipette tip 70A-B or inward from an inner surface 157 at the distal end of the pipette tip 70C. (Also contemplated by the term "rib structures", as applied to any example herein, is a series of abbreviated or interrupted rib structures (not shown) which, for example, may be in the form of a series of protuberances which are the same or different in size and shape and which are equally or unequally spaced apart.) The addition of these lower rib structures 151A-C, 152A-C was found to strengthen the pipette tip 70A-C so that it can more easily penetrate the cap 20A-C without bending. Bending of the pipette tip 70A-C could prevent penetration of the cap 20A-C, occlude an orifice 161 of the pipette tip 70A-C and/or misdirect a fluid stream subsequently dispensed from the pipette tip 70A-C.

While the lower rib structures 151A-B, 152A-B can be placed or integrated along any substantially vertical line (or positioned in any other orientation which increases the rigidity of the pipette tip 70A-C but does not interfere with its functions of penetrating a surface material and drawing or dispensing fluids) on the outer surface 153 of the pipette tip 70A-B, it is generally desirable to have at least one lower rib structure 151A positioned on the outer surface 153 at the distal end of the pipette tip 70A so that a terminus 162A of the lower rib structure 151A co-terminates with the point 155A of a beveled tip 71A. (It is noted that lower rib structures 151A-C, 152A-C can also be used with pipette tips which have a flat or blunt-ended surface surrounding the orifice 161 at the distal end (not shown).) If the pipette tip 70A-B includes more than one lower rib structure, then the lower rib structures 151A-B, 152A-B are preferably circumferentially spaced-apart at equal distances on the outer surface 153 at the distal end of the pipette tip 70A-B, although this precise arrangement of lower rib structures 151A-B, 152A-B is not a requirement.

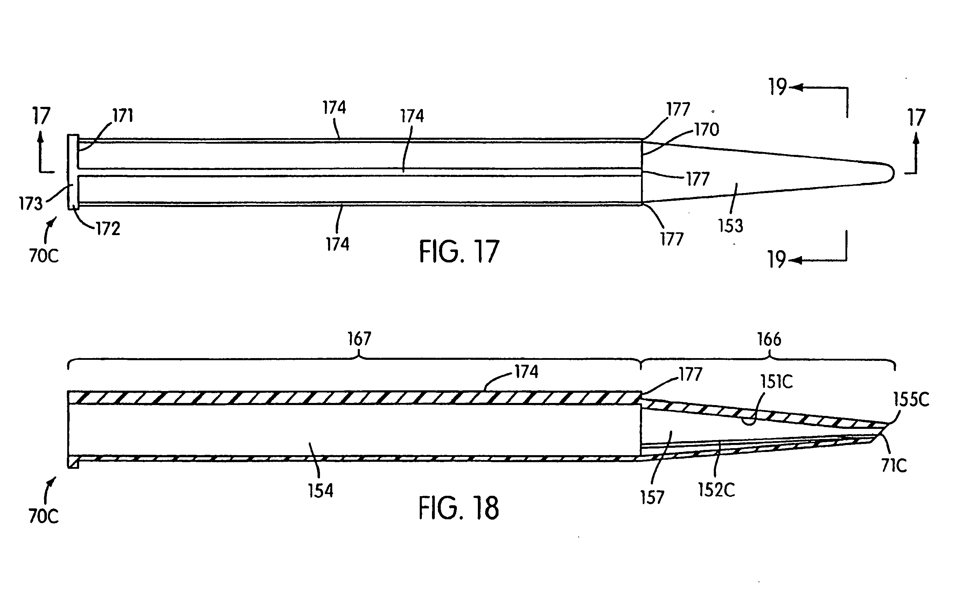

Ideally, the pipette tip 70A-C is a conventional single-piece, plastic pipette tip modified to include the lower rib structures 151A-C, 152A-C during manufacture using any wall-known injection molding procedure. Alternatively, the lower rib structures 151A-C, 152A-C may be applied to the outer or inner surface 153, 157 of the pipette tip 70A-C using, for example, an inert adhesive. An example of an acceptable pipette tip, prior to any of the modifications described herein, is an ART® 1000 µl pipette tip available from Molecular BioProducts of San Diego, CA as Cat. No. 904-011. This particular pipette tip is especially preferred for applications where carryover contamination is a concern, since it includes a filter (not shown) located at a position within an interior chamber 154 of the pipette tip 70A-C *(see* FIG. 18) which functions to block or impede the passage of potentially contaminating liquids or aerosols generated during pipetting. While the preferred number of lower rib structures 151A-C, 152A-C is three, the precise number selected should be determined, at least in part, by the type of resin or combination of resins used to manufacture the pipette tip 70A-C as well as the expected force needed to pierce a penetrable cap 20A-C or other surface material, when such is an intended use of the pipette tip 70A-C. Where a softer material is chosen for manufacturing the pipette tip 70A-C, or more force will be required to pierce a surface, it may be desirable to increase the number of lower rib structures 151A-C, 152A-C on the pipette tip 70A-C.

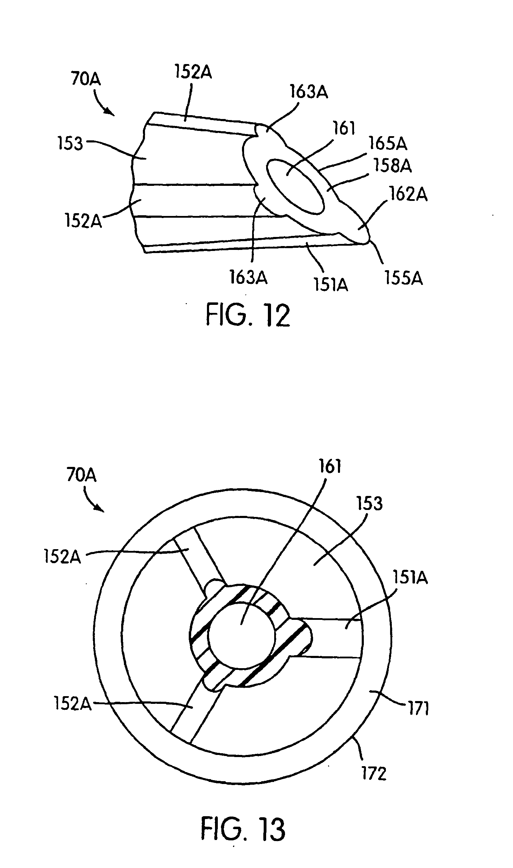

Another means by which to increase the rigidity of the pipette tip 70A-C is to adjust the thickness or width of the lower rib structures 151A-C, 152A-C. In an example, the lower rib structure 151A which co-terminates with the beveled tip 71A has a greater thickness and width than any of the other lower rib structures 152A positioned on the pipette tip 70A. As shown in Figs. 12 and 13, the larger of these preferred lower rib structures 151A substantially forms a semi-circle in cross-section having a radius of about 0.020 inches (0.508 mm), whereas each of the smaller preferred lower rib structures 152A, which also substantially form semi-circles in cross-section, has a radius of about 0.012 inches (0.305 mm) in this example. Of course, those skilled in the art will be able to readily adjust the thicknesses and depths of the lower rib structures 151A-C, 152A-C by taking into consideration the properties of the resin selected and the anticipated force needed to penetrate one or more pre-selected surface materials. And although the shape of the preferred lower rib structures 151A-C, 152A-C is substantially a solid semi-circle in cross-section, the lower rib structures 151A-C, 152A-C of the present example may have either a solid or hollow core and can be constructed to include any one or a combination of geometric and/or non-geometric shapes (in cross-section), provided the shape or shapes of the lower rib structures 151A-C, 152A-C do not significantly interfere with the penetration or fluid-flow characteristics of the pipette tip 70A-C. Examples of approximate geometric shapes which may be used for the lower rib structures 151A-C, 152A-C include, but are not limited to, a triangle, a square, a rectangle, a semi-circle, and a nearly complete circle.

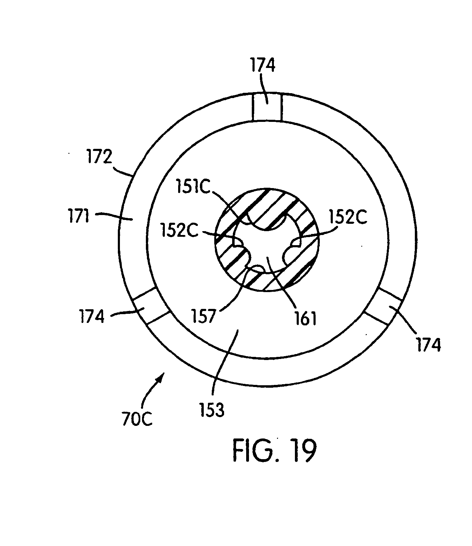

Although the preferred location of the lower rib structures 151A-B, 152A-B is on the outer surface 153 at the distal end of the pipette tip 70A-B, positioning the lower rib structures on the inner surface 157 at the proximal end of the pipette tip 70C may have certain advantages. For instance, positioning the lower rib structures 151C, 152C on the inner surface 157 of the pipette tip 70C could simplify the injection molding procedure by making it easier and less costly to prepare the molds. Additionally, positioning the lower rib structures 151C, 152C on the inner surface 157 may reduce the formation or extent of hanging drops on the bottom surface (not shown) of the pipette tip 70C and reduce the adherence of fluid to the outer surface 153 of the pipette tip 70C by reducing the surface area of the pipette tip 70C which comes into contact with a fluid. In this particular configuration, the lower rib structures 151A, 152A shown in Figs. 10 and 11 could be positioned in a mirrored fashion on the inside of the cone section 166, as shown in FIG. 18, being careful to choose thicknesses for these internally positioned lower rib structures 151C, 152C, and adjusting the size of an orifice 161 at the distal end of the pipette tip 70C, so that the movement of fluids into or out of the pipette tip 70C will not be substantially impeded. One possible arrangement designed to avoid excessive disruption of the flow of fluids into or out of the pipette tip 70C is shown in cross-section in FIG. 19. Determining appropriate dimensions for these internal, lower rib structures 151C, 152C and the orifice 161 size of the pipette tip 70C would require nothing more than routine experimentation and would depend upon the particular application.

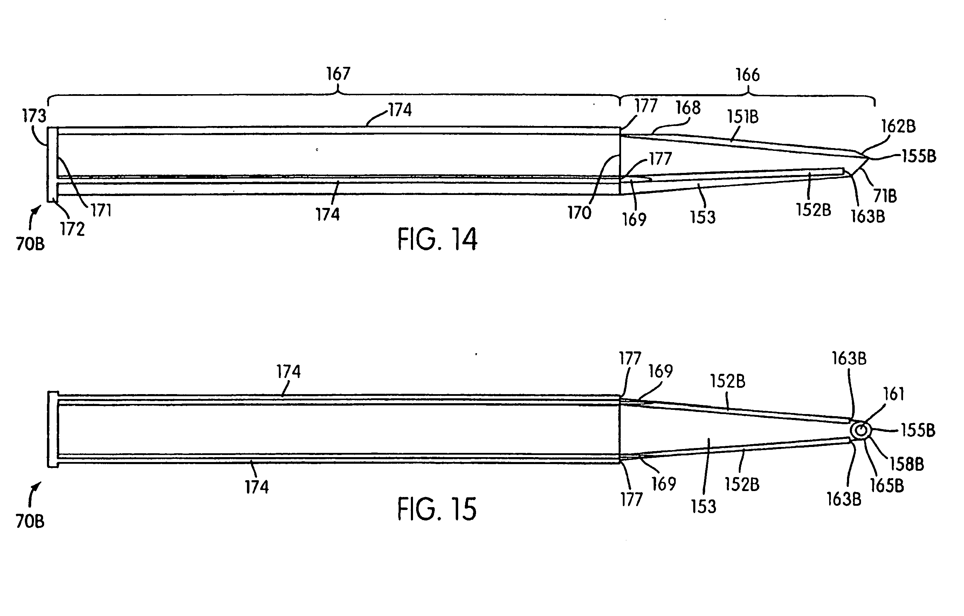

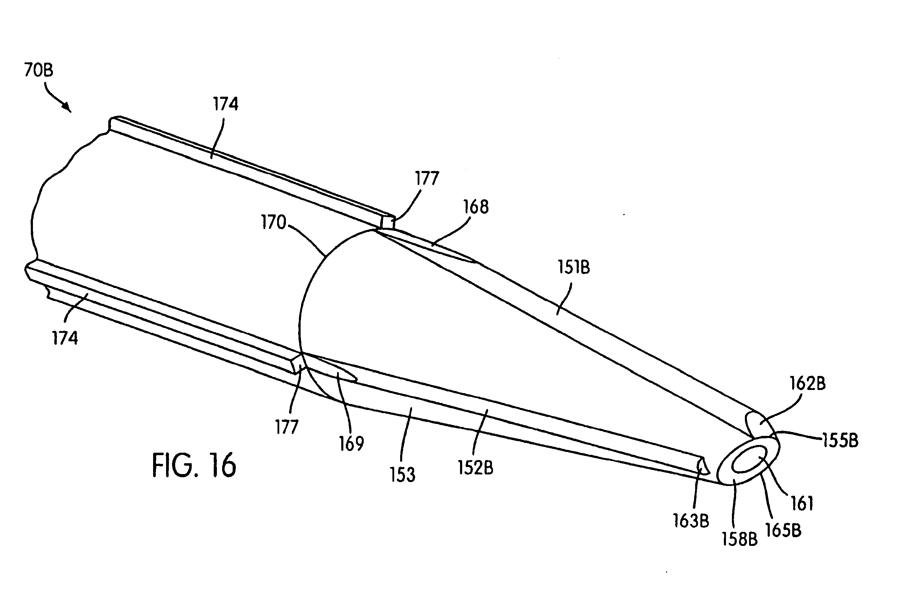

The preferred distal termini 162A, 163A of the lower rib structures 151A, 152A, as shown in FIG. 12, are flush with and partially define the bottom surface 158A at the distal end of the pipette tip 70A. Thus, when the pipette tip 70A has a beveled tip 71A, as depicted in Figs. 10-12, the distal terminus 162A, 163A of each of the lower rib structures 151A, 152A will share the same angle as the beveled tip 71A with respect to the axis of symmetry 72 shown in FIG. 10. In the preferred pipette tip 70A, this angle is about 30° to about 60°, more preferably about 35° to about 55°, and most preferably 45° ± 5°. However, it is not a requirement that the distal termini 162A, 163A be flush with and partially define the bottom surface 158A of the pipette tip 70A. For example, Figs. 14 and 16 highlight an alternative configuration where the distal terminus 162B of the rib structure 151B tapers away from (rather than forms) a point 155B of the beveled tip 156B, thus creating more of a wedge-like shape to the point 155B of the pipette tip 70B. As Figs. 14-16 show, the lower rib structures 151B, 152B can also be positioned so that the surfaces of the distal termini 162B, 163B are not co-extensive with the bottom surface 158B at the distal end of the pipette tip 70B, but are instead formed at a point vertically above the bottom surface 158B. (While only the smaller of the lower rib structures 152B is actually depicted in this manner in Figs. 14-16, the distal terminus 162B of the larger of the lower rib structures 151B could likewise be positioned above the bottom surface 158B.) Decreasing the surface area of the bottom surface 158B, in a manner similar to that shown in FIG. 16, could be advantageous if it is desirable to minimize fluid droplet formation at the distal end of the pipette tip 70B due to surface tension.

While the distal termini 163B of the lower rib structures 152B shown in Figs. 14-16 are blunt-ended, alternative designs could be equally acceptable. As an example, the smaller lower rib structures 152B could have a tapered shape similar to that shown in FIG. 14 for the larger lower rib structure 151B. A tapered form of the smaller lower rib structure 152B might terminate at the outer circumference 165B of the bottom surface 158B shown in Figs. 15 and 16 or at some point above the bottom surface 158B. Whatever shape or terminus location is selected for each lower rib structure 151A-C, 152A-C, the primary considerations in most cases will be the effect that the size, shape, number and positioning of the lower rib structures 151A-C, 152A-C have on the force needed to penetrate a surface material and the resulting strength of the pipette tip 70A-C.

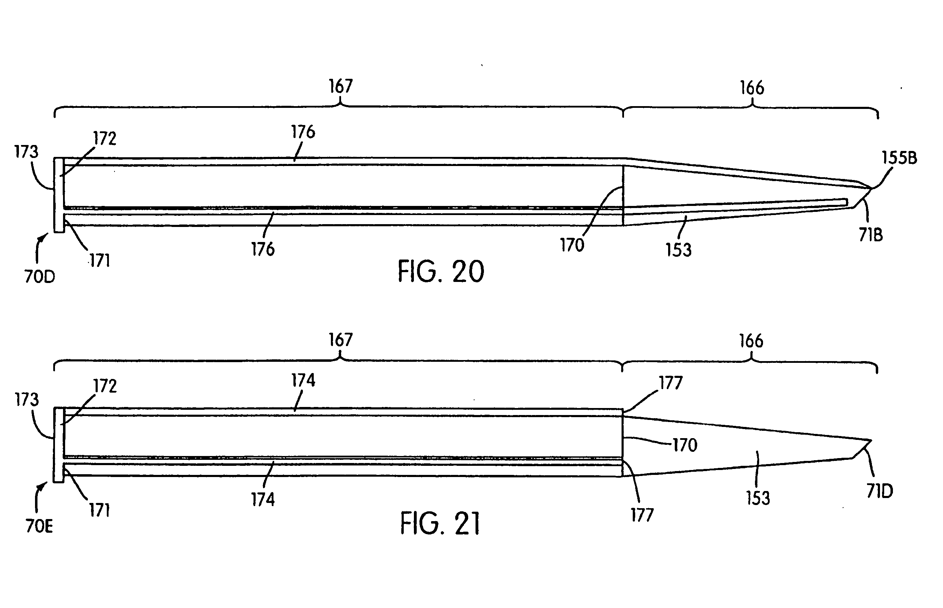

The distance that the preferred lower rib structures 151A-B, 152A-B extend away from the distal termini 162A-B, 163A-B, which generally will be located at or near the bottom surface 158A-B of the pipette tip 70A-B, may vary between lower rib structures 151A-B, 152A-B on the same pipette tip 70A-B and may be of any length, although preferred lengths are at least about 0.25 inches (6.35 mm), at least about 0.5 inches (12.7 mm), and at least about 1.0 inch (25.4 mm). Where the distal termini 162A-B, 163A-B are located "near" the bottom surface 158A, 158B, the distance from an outer perimeter 165A, 165B at the distal end of the pipette tip 70A-B to each distal terminus 162A-B, 163A-B is no more than about 0.5 inches (12.7 mm), and preferably no more than about 0.25 inches (6.35 mm) (this definition of "near" is equally applicable to descriptions of the distal termini (not shown) of lower rib structures 151C, 152C positioned on the inner surface 157 of the cone section 166 and the continuous rib structures 176 described *infra*). As illustrated in Figs. 10, 11, 14 and 15, the pipette tip 70A-B forms a cone section 166 at the distal end of the pipette tip 70A-B, and the lower rib structures 151A-B, 152A-B extend from or near the bottom surface 158A-B of the pipette tip 70A-B to a point at the proximal end of the cone section 166, where the cone section 166 converges with a tube section 167. In this example, the proximal terminus 168, 169 of each lower rib structure 151A-B, 152A-B tapers to a point where it meets the circumferential line 170 separating the cone section 166 from the tube section 167. The lower rib structures 151A-B, 152A-B may also extend from a point at or near the bottom surface 158A-B to any point on the tube section 167, even to a point at or near a top surface 173 at the proximal end of the pipette tip 70A-B (if no flange 172 is present) or, as shown in FIG. 20, a bottom surface 171 of the flange 172 at the proximal end of the pipette tip 70D.

By extending the lower rib structures 151A, 152A to a point or points on the tube section 167, (*see, e*.*g*., FIG. 20), or separately or exclusively positioning upper rib structures 174 on the tube section 167, *(see* Figs. 14-18 for examples of "separate" positioning and FIG. 21 for an example of "exclusive" positioning), benefits are expected to inhere when the pipette tip 70B-E will be used to penetrate a surface material associated with a fluid-containing vessel 50. The most important of these benefits is the creation of air gaps or passageways that permit at least a portion of the volume of air being displaced by entry of the pipette tip 70B-E into the vessel 50 of the collection device 10 to escape through openings in the punctured surface material. Upon surface penetration, these passageways form in areas adjacent to the contact points between the upper rib structures 174 or continuous rib structures 176 and the penetrated surface material. By creating these passageways during penetration, the upper rib structures 174 and continuous rib structures 176 aid in preventing a high pressured movement of air through openings in the penetrated surface material as the pipette tip 70B-E is being inserted into or withdrawn from the collection device 10.

With fluid transfer devices having smaller diameters, such as fluid-transporting needles, air displacement by the fluid transfer device entering a collection device may be less of a concern. Notwithstanding, there may still be concerns about pressure differences between the interior space of the collection device and the surrounding environment. When the air pressure inside of the collection device is sufficiently greater than the ambient air pressure, then there is a risk that at least some of the fluid material inside of the collection device will escape through the opening created in a penetrated surface material when the fluid transfer device is withdrawn from the collection device. This is because the penetrated surface material may form a seal around the entering fluid transfer device which is largely broken when the fluid transfer device is completely withdrawn from the collection device, at which time fluid material in the form of aerosols or bubbles may escape from the collection device as the two air pressures rapidly seek equilibrium. Moreover, because the penetrated surface material may form a seal around the fluid transfer device, a partial vacuum within the collection device may be created which could draw fluid material out of the fluid transfer device, thereby affecting pipetting accuracies and possibly leading to dripping of fluid material as the fluid transfer device is withdrawn from the collection device. To minimize or eliminate these potential problems, it is important to provide a passageway for venting air from the collection device as the surface material is being penetrated by the fluid transfer device and to maintain this passageway as the fluid transfer device is withdrawn. This can be achieved by adding upper or continuous rib structures 174, 176 to at least some portion of the fluid transfer device expected to be in contact with the surface material to be penetrated by the fluid transfer device as it enters the collection device to remove fluid material therefrom. In this way, small air gaps will be created between the penetrated surface material and a portion of the fluid transfer device, thereby facilitating equilibrium between the interior and exterior air pressures before the fluid transfer device is fully withdrawn from the collection device.

Where the upper rib structures 174 are distinct from the lower rib structures 151B, 152B, as shown in Figs. 14-16, the upper rib structures 174 are preferably aligned in tandem with an equal number of lower rib structures 151B, 152B positioned in a generally vertical orientation. The upper rib structures 174 are preferably integrally molded with the tube section 167 using any well known injection molding process, however, the upper rib structures 174 might be applied to the tube section 167 using, for example, an inert adhesive. While even one upper rib structure 174 can provide a beneficial air gap, the preferred number of upper rib structures 174 is at least three. There is, however, no set limit on the number of upper rib structures 174 that may be positioned on the tube section 167. But where at least one purpose of the upper rib structures 174 is to vent the interior chamber 175 of the collection device 10, then the size, shape, number and orientation of the upper rib structures 174 should be chosen so that air gaps will be formed during pipetting, thus facilitating adequate venting of displaced air and/or the equilibration of air pressures inside and outside of the collection device 10.

As with the lower rib structures 151A-C, 152A-C, the upper rib structures 174 may be of any one or a combination of geometric and/or non-geometric shapes, when viewed in cross-section, provided the shape or shapes of the upper rib structures 174 do not significantly interfere with the penetration characteristics of the pipette tip 70B-E which incorporates them. The shapes of the upper rib structures 174, when used in conjunction with lower rib structures 151A-C, 152A-C, may be the same or different than the shapes of the lower rib structures 151A-C, 152A-C. Examples of possible shapes include semi-circles, nearly complete circles, triangles, squares and rectangles. Preferably, the cross-sectional shape of each upper rib structure 174 is a square measuring about 0.02 inches (0.508 mm) in width by about 0.02 inches (0.508 mm) in height (measuring from the outer surface 153 of the tube section 167). The precise dimensions of the upper rib structures 174 are not critical, provided the upper rib structures 174 are capable of producing the desired air gaps without significantly interfering with the penetration characteristics of the pipette tip 70B-E.

As indicated above, the lower and upper rib structures of the pipette tip 70D may form continuous rib structures 176, as shown in FIG. 20, thereby creating rib structures 176 which are unbroken between the cone and tube sections 166, 167. Notwithstanding, the preferred pipette tip 70B incorporates distinct lower and upper rib structures 151B, 152B, 174. In this example, which is depicted in Figs. 14-16, the lower rib structures 151B, 152B taper at their proximal ends to form termini 168, 169, which terminate at the circumferential line 170 delineating the cone and tube sections 166, 167. The upper rib structures 174 in this preferred mode have blunt-ended termini 177 at their distal ends which terminate at the circumferential line 170, although the upper rib structures 174 could likewise taper in a mirrored fashion to lower rib structures 151B, 152B, terminating at the circumferential line 170.

To further facilitate penetration of the cap 20A-C, the fluid transfer devices 70, 70A-E preferably include a beveled tip 71, 71A-D as shown in Figs. 7, 10, 12, 14, 16, 18, 20 and 21. When a beveled tip 71, 71A-D is employed, the distal end of the fluid transfer device 70, 70A-E (e.g., fluid-transporting needle or pipette made of a resin) preferably has an angle of about 30° to about 50° with respect to the axis of symmetry 72 of the fluid transfer device 70, 70A-E. Most preferably, the angle of the beveled tip 71, 71 A-E is about 45° ± 5° with respect to the axis of symmetry 72 of the fluid transfer device 70, 70A-E, as shown in Figure 7. However, a beveled tip of any angle that improves the penetrability of a cap is desirable, provided the integrity of the fluid transfer device is not compromised when the tip penetrates the cap, thereby affecting the ability of the fluid transfer device to predictably and reliably dispense or draw fluids.

In order to be useful, the fluid transfer devices used in connection with the present invention should be constructed so that their proximal ends can be securely engaged by a probe associated with an automated or manually operated fluid transfer apparatus. A fluid transfer apparatus is a device which facilitates the movement of fluids into or out of a fluid transfer device, such as a pipette tip. An example of an automated fluid transfer apparatus would be a GENESIS Series Robotic Sample Processor available from TECAN AG of Hombrechtikan, Switzerland, and an example of a manually operated fluid transfer apparatus is the Pipet-Plus® Latch-Mode^{™} Pipette available from the Rainin Instrument Company of Emeryville, CA.

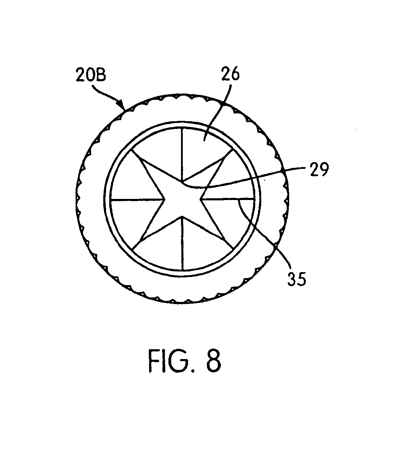

Returning to the description of the conical inner wall 33 depicted in various embodiments in Figs. 1-9, it should be pointed out that the number of striations 35 selected and the distance that those striations 35 extend from start-points 31 at or near the apex 34 to the outer circumference 38 of the conical inner wall 33 should be sufficient to maintain at least a portion of the generally wedge-shaped sections 26 of the conical inner wall 33 in an "open" configuration after the conical inner wall 33 has been penetrated by a fluid transfer device 70 and the fluid transfer device 70 has been removed from the cap 20A-C. As illustrated in FIG. 8, the wedge-shaped sections 26 of the conical inner wall 33 are in an "open" configuration provided that at least a portion of the tips 29 of the wedge-shaped sections 26 are not in physical contact with one another after the fluid transfer device 70 has been removed from the cap 20A-C. (The conical inner wall 33 is deemed to be in the "open" configuration when at least two of the wedge-shaped sections have separated from one another after penetration of the cap 20A-C by the fluid transfer device 70.) By maintaining the wedge-shaped sections 26 in an "open" configuration, frictional contact between the cap 20A-C and fluid transfer device 70 is reduced and venting of air inside of the collection device 10 is facilitated.

The distance that the striations 35 extend from the apex 34, or start-points 31 near the apex 34, of the conical inner wall 33 to the outer circumference 38 of the conical inner wall 33 may be any distance sufficient to improve the penetrability of the conical inner wall 33 as compared to an identical conical inner wall 33 having no striations 35. An improvement in penetrability is measured as a reduction in the force required to penetrate the conical inner wall 33 of the cap 20A-C, as described hereinabove. While it is not essential that all of the striations 35 extend the same distance, it is preferred that each striation 35 extend radially outwardly at least about a quarter the distance from the apex 34, or a start-point 31 near the apex 34, to the outer circumference 38 of the conical inner wall 33. In a more preferred mode, each striation 35 extends radially outwardly at least about half the distance from the apex 34, or start-points 31 near the apex 34, to the outer circumference 38 of the conical inner wall 33. And in the most preferred embodiment of the present invention, each striation 35 extends radially outwardly from the apex 34, or a start-point 31 near the apex 34, to the outer circumference 38 of the conical inner wall 33.

Another factor to be considered in determining what distance the striations 35 should extend from the apex 34 to the outer circumference 38 of the conical inner wall 33 is the circumferential size of the fluid transfer device. As the circumferential size of the fluid transfer device increases, the distance that the striations 35 extend from the apex 34 or start-points 31 near the apex 34 to the outer circumference 38 of the conical inner wall 33 will likewise need to increase in order to improve penetration, allow for the formation of adequate air passageways, and to minimize the frictional forces applied to fluid transfer device by the conical inner wall 33 when the fluid transfer device is entering and being withdrawn from the collection device 10. Increasing the number of striations 35 will also aid in reducing the frictional forces applied by the conical inner wall 33.

Because the striations 35 may be formed as channels, grooves, etchings or a series of perforations in the conical inner wall 33, the thicknesses of the striations 35 present in the conical inner wall 33 -- which may be the same or different from one another -- are less than the thicknesses of the surrounding areas the conical inner wall 33. When determining the different thicknesses of a conical inner wall 33, the cap 20A-C should first be cooled at room temperature for a period of at least one hour after forming, or cooled in tap water for at least 10 to 15 minutes, so that the resin can sufficiently harden. Four sections of the cap 20A-C, each preferably including a different striation 35 in cross-section, may then be cut at right angles to the striations 35 using an exacto or utility knife. With each of these sectional pieces of the conical inner wall 33 of the cap 20A-C, a single measurement can be taken from each of the striated and non-striated portions using any sensitive measuring means, including calipers and/or video-based measuring instruments, in order to determine the thicknesses between the inner and outer surfaces 36, 37 of the conical inner wall 33 in these portions. For the striated portions, the thickness measurements should be based on the smallest cross-sectional thickness between the inner and outer surfaces 36, 37. The thickness values thus obtained can be averaged to calculate the approximate thicknesses of the striated and non-striated portions making up the conical inner wall 35 of the cap 20A-C.

In a further example, the thickness ratio, which is based on the ratio of the average thickness of the non-striated portions of the conical inner wall 33 to the average thickness of the striations 35 in the conical inner wall 33, is preferably in the range of about 5:1 to about 1.25:1, more preferably in the range of about 7.5:1 to about 2:1, and most preferably in the range of about 10:1 to about 2.5:1. The average thickness of the striations 35 of the conical inner wall 33 is preferably in the range of about 0.002 inches (0.051 mm) to about 0.008 inches (0.203 mm), and the average thickness of the surrounding areas of the conical inner wall 33 is preferably in the range of about 0.01 inches (0.254 mm) to about 0.02 inches (0.508 mm). (The indicated thicknesses for the striations are also the preferred thicknesses of the conical inner 33 when no striations 35 are included.) More preferably, the average thickness of the surrounding areas of the conical inner wall 33 is about 0.010 inches (0.254 mm) to about 0.017 inches (0.432 mm); about 0.012 inches (0.305 mm) to about 0.015 inches (0.381 mm); and about 0.013 inches (0.330 mm). At a minimum, the difference in average thicknesses between the striations 35 and the surrounding areas of the conical inner wall 33 should be such that the resistance encountered by the fluid transfer device as it passes through the conical inner wall 33 is less than it would be in the absence of such striations 35, *i*.*e*, a conical inner wall 33 having a substantially uniform thickness.

When the striations 35 include a series of perforations, the perforations are preferably sized to limit or prevent the passage of fluid substance in the vessel 50 to the inner surface 36 of the conical inner wall 33, where it could come into contact with a practitioner. This is particularly important where the fluid substance contains a potentially contaminating material (*e*.*g*., pathogenic organism). To further ensure that no contaminating contact occurs between a practitioner and a fluid substance contained in the vessel 50 of the collection device 10 when perforations constitute part or all of the striations 35 in the conical inner wall 33, the seal 80 discussed hereinabove may be applied to the upper surface 24 of the annular top wall 22 (cap 20A-B) or to the annular top surface 48 (cap 20C) during manufacture so that the aperture leading to the conical inner wall 33 remains completely enclosed.

Nonetheless, even when a seal 80 is employed, series of perforations do not constitute the preferred striations 35 used in an embodiment of the present invention. This is especially the case where the collection device 10 will be shipped and potentially exposed to fluctuations in temperature and pressure which could result in fluid material leaking through the perforations, particularly where the collection device 10 is not expected to remain upright during shipping. Additionally, fluid which has leaked through perforations present in the conical inner wall 33 to the inner surface 36 could be absorbed by an optionally present wick 90, possibly causing the wick 90 to become saturated. Insertion of a fluid transfer device through a wick 90 so effected may actually promote aerosol formation and/or bubbling and, thus, the spread of potential contaminants. Accordingly, the use of series of perforations for the striations 35 is not recommended except when it is certain the collection devices 10 will remain upright and will not be exposed to unacceptable changes in temperature and pressure.

As shown in Figs. 5 and 6, the annular outer flange 40, 40A has an inner surface 41, 41A adapted to grip an upper portion 62 (*see* FIG. 1) of the outer surface 53 of the vessel 50, such that an essentially leak-proof seal between the cap 20A-C and the vessel 50 can established. More specifically, the essentially leak-proof seal may be created between the lower surface 23 of the annular top wall 22, 22A of the cap 20A-C and the upper surface 52 of the annular rim 51 of the vessel 50. Under normal handling conditions, this essentially leak-proof seal will prevent seeping of specimen from an interior chamber 175 of the vessel 50 to an area of the outer surface 53 of the vessel 50 which might be contacted by a practitioner during routine handling. Normal handling conditions would exclude the application of excessive and unusual forces (*i*.*e*., forces sufficient to puncture or crush a cap or vessel), as well as temperature and pressure fluctuations not typically experienced in the handling and transport of collection devices.

The inner surface 41 of the annular outer flange 40 may be adapted, as depicted in FIG. 5, to include a thread 42, which permits the cap 20A-C to be screwed onto an upper portion 62 of the outer surface 53 of the vessel 50 *(see* FIG. 1), where the vessel 50 has a mated thread 54. The mated threads 42, 54 facilitate an interlocking contact between the thread 42 of the cap 20A-B and the thread 54 of the vessel 50. Screw-type caps are well known in the art and skilled practitioners will readily appreciate acceptable dimensions and means of manufacture. Ideally, the threads 42, 54 are integrally molded with the cap 20A-C and the vessel 50, respectively.

Another adaption to the inner surface 41A of the annular outer flange 40A contemplated in connection with the present invention is a snapping structure, as illustrated in FIG. 6. Here, the inner surface 41A of the annular outer flange 40A is adapted to include a rim 43 which can be snapped over a mated rim 55 on the outer surface 53 of the upper portion 62 of the vessel 50 (*see* FIG. 1). These rims 43, 55 are preferably integrally molded with the annular outer flange 40A of the cap 20C and the outer surface 53 of the vessel 50, respectively. In order to create this snapping feature, the materials selected for constructing the cap 20C and vessel 50 must be sufficiently resilient and the diameter of the inner portion 45 of the rim 43 on the cap 20C must be sized to be less than the diameter of the outer portion 56 of the rim 55 on the vessel 50, so that the inner portion 45 of the rim 43 on the cap 20C, as defined by the circumference of the inner portion 45 of the rim 43, can fit over the outer portion 56 of the rim 55 on the vessel 50, as defined by the circumference of the outer portion 56 of the rim 55, without requiring the application of a mechanical force. Moreover, the location of the rims 43, 55 should be such that the lower portion 57 of the rim 55 on the vessel 50 nests in an overlapping fashion on the upper portion 44 of the rim 43 of the cap 20C after the cap 20C has been fitted onto the vessel 50. Moreover, when the rim 55 of the vessel 50 is nesting on the rim 43 of the cap 20C, an essentially leak-proof seal should be formed between the lower surface 23 of the annular top wall 22A of the cap 20C and the upper surface 52 of the annular rim 51 of the vessel 50.

Regardless of the method adopted for physically and sealably associating the cap 20A-C and vessel 50, the essentially leak-proof nature of this arrangement can be further improved by including two simple modifications to the cap 20A-C, as illustrated in Figs. 5 and 6. The first modification would be to create an angled portion 47 on the inner surface 41, 41 A of the annular outer flange 40, 40A at the point where the annular rim 51 of the vessel 50 and the annular outer flange 40, 40A make contact. In this way, the frictional contact between the angled portion 47 of the inner surface 41, 41A and the annular rim 51 of the vessel 50 will create a more secure barrier to the passage of fluids from within the vessel 50. (The space shown in these figures between the lower surface 23 of the annular top wall 22, 22A of the cap 20A-C and the upper surface 52 of the rim 51 of the vessel 50 would be non-existent or less severe when the cap 20A-C is securely fitted onto the vessel 50.) Additionally, the outer circumference 38 of the conical inner wall 33 can be modified to include an annular outer rim 39 (*see* FIG. 5) or annular skirt 12I (*see* FIG. 6) which is designed to be in frictional contact with the inner surface 59 of the side wall 58 of the vessel 50 when the cap 20A-C and vessel 50 are physically and sealably associated. Contact between the inner surface 59 of the side wall 58 and either the annular outer rim 39 or an outer wall 122 of the annular skirt 121 should further impede the leaking of fluids from the vessel 50.

An alternative to the annular outer flange 40, 40A described hereinabove would be an annular flange (not shown) having an outer surface adapted to grip the inner surface 59 of the side wall 58 within the open-ended, upper portion 62 of the vessel 50. Such an annular flange could be constructed to frictionally fit within the upper portion 62 of the vessel 50 in a manner similar to that described above for gripping the outer surface 53 of the upper portion 62 of the vessel 50 with the inner surface 41, 41 of the annular outer flange 40, 40A. In another form, the annular flange could be sized to fit snugly within the upper portion 62 of the vessel 50 without the need to include a rim or thread on both the outer surface of the annular flange and the inner surface 59 of the vessel 50. In all other respects, this cap could be designed to include the features described herein for the cap 20A-C, including a wick 90 and/or seal 80. It is also possible to remove the annular outer flange 40, 40A altogether, thereby converting the annular top wall 22 into an annular ring (not specifically shown) having a lower surface which can be affixed to the upper surface 52 of the annular rim 51 of the vessel 50 using, for example, an adhesive (e.g., an inert glue).

To improve the seal formed between the annular rim 51 of the vessel 50 and the lower surface 23 of the annular top wall 22, 22A of the cap 20A-C when the vessel 50 and cap 20A-C are in fixed association, an annular seal (not shown) in the shape of an O-ring may be sized to fixedly nest on the lower surface 23 of the annular top wall 22, 22A. The annular seal may be an elastomeric material (*e.g*., neoprene) whose thickness is chosen so that snapping of the rim 43 of the cap 20C over the rim 55 of the vessel 50, or screwing the cap 20A-B onto the vessel 50 so that their respective threads 42, 54 are interlocking, is not prevented.

### EXAMPLE

To determine the amount of force needed to penetrate a cap 20A-C being part of the present invention, a Universal Tension/Compression Tester ("Compression Tester"), Model No. TCD 200, and a force gauge, Model No. DFGS-50, were obtained from John Chatillon & Sons, Inc. of Greensboro, N.C. Because the Compression Tester is an automated instrument, it allows for greater reproducibility when determining the compression needed to penetrate a cap that may not be possible following a purely manual approach.

All caps 20A-C used in this test were made of HDPE and had a substantially uniform thickness of between about 0.0109 inches (0.277 mm) and about 0.0140 inches (0.356 mm), except in the region of the striations 35. The depth of the conical inner wall 33 of the cap 20A-C was about 0.29 inches (7.37 mm) as measured along the axis of symmetry 30 of the annular top wall 22, 22A from the plane of the outer circumference 38 of the conical inner wall 33 and moving to the apex 34 of the same. The diameter of the outer circumference 38 of the conical inner wall 33 was about 0.565 inches (14.35 mm). With all caps 20A-C tested, the conical inner wall 33 had a single angle of about 35° or about 45° from the axis of symmetry 30 of the annular top wall 22, 22A.

When caps 20A-C being tested included striations 35, the thickness of the conical inner wall 33 at the approximate center of each striation 35 was in the range of about 0.0045 inches (0.114 mm) to about 0.0070 inches (0.178 mm), where all striations 35 of any given cap 20A-C were of substantially the same thickness and had an approximate width of 0.015 inches (0.381 mm). The total number of striations 35 for striated caps 20A-C was always eight and the striations 35 were all formed on the inner surface 36 of the conical inner wall 33 during the injection molding process. Striations 35 of the caps 20A-C tested extended either fully or about half the distance from or near the apex 34 to the outer circumference 38 of the conical inner wall 33.

The caps 20A-C were threadingly secured to a vessel 50 measuring approximately 13 mm x 82 mm and made of polypropylene. In order to stabilize the vessels 50 prior to penetration with the force gauge, each vessel 50 was secured in an aluminum block having a hole bored therein for receiving and stably holding the vessel 50. The precise method chosen for positioning a collection device 10 under the force gauge is not critical, provided the collection device 10 is secured in a vertical position under the force gauge, as judged by the axis of symmetry 30.

In evaluating the force required to penetrate a cap 20A-C, the vessel 50 with attached cap 20A-C was first centered under the force gauge with a Genesis series 1000 µl Tecan-Tip pipette tip force-fitted onto a 2 inch (50.8 mm) extension located at the base of the force gauge. The pipette tips were either blunt-ended or beveled with an angle of about 45° at their distal ends. A cap 20A-C was considered to be centered when the pipette tip was located above the apex 34 of the conical inner wall 33 of the cap 20A-C. Absolute centering was not essential since the shape of the conical inner wall 33 of the cap 20A-C naturally directed the pipette tip to the apex 34 of the conical inner wall 33 of the cap 20A-C. Since the pipette tip moved at a constant rate of 11.25 inches (285.75 mm)/minute, the initial height of the pipette tip above the cap 20A-C was not critical, provided there was some clearance between the cap 20A-C and the pipette tip. For testing purposes, however, the pipette tip was generally positioned at least about 0.2 inches (5.08 mm) above the upper surface 24, 24A of the annular top wall 22, 22A and permitted to penetrate up to 2.8 inches (71.12 mm) into the vessel 50, thereby avoiding actual contact with the inner surface 61 of the bottom wall 60 of the vessel 50. The penetration force required was measured in pounds, and for all cap 20A-C tested the penetration force was less than about 6.5 pounds (2.95 kg). With fully-striated cap 20A-C and beveled pipette tips, the penetration force was generally less than about 4.0 pounds (1.81 kg), and in some cases the penetration force required was about 3.6 pounds (1.63 kg) or less.

While the present invention has been described and shown in considerable detail with reference to certain preferred embodiments, those skilled in the art will readily appreciate other embodiments of the present invention. Accordingly, the present invention is deemed to include all modifications and variations encompassed within scope of the following appended claims.

## Claims

1. A sealed collection device (10) comprising:
a vessel (50) having a side wall (58) with an inner surface (59),
a cap (20) closing the sealed collection device and having an inner wall (33) with an outer surface (37) extending into the vessel opposed to the inner surface (59) of the side wall (58) of the vessel (50); and
a specimen retrieval device (130) extending within the vessel (50),
the cap (20) being pierceable along a fluid transfer pathway of a fluid transfer device (70), the pathway extending through the cap (20) and into the vessel (50); and the specimen retrieval device (130) extending along the inner surface (59) of the side wall (58) of the vessel (50)
**characterized in that** one end of the specimen retrieval device (130) is fitted between the outer surface (37) of the inner wall (33) of the cap (20) and the opposed inner surface (59) of the side wall (58) of the vessel (50) such that the specimen retrieval device (130) is sufficiently isolated from the pathway of the fluid transfer device (70).

2. The sealed collection device (10) of claim 1, wherein the inner wall (33) of the cap (20) has a generally conical shape.

3. The sealed collection device (10) of claim 1 or 2 further comprising a penetrable seal (80) covering an opening in the cap (20) above the inner wall (33) of the cap (20).

4. The sealed collection device (10) of claim 3, the cap further comprising a wick (90) positioned between the inner wall (33) of the cap (20) and the seal (80), the wick (90) being adapted to inhibit the release of aerosols from the collection device (10).

5. The sealed collection device (10) of claim 3 or 4, wherein the seal (80) comprises a metallic foil.

6. The sealed collection device (10) of claim 3 or 4, wherein the seal (80) comprises a plastic.

7. The sealed collection device (10) of any one of claims 1 to 6, wherein the cap (20) comprises a molded plastic.

8. The sealed collection device (10) of any one of claims 1 to 7, wherein the inner wall (33) of the cap (20) is a molded plastic comprising a plurality of striations (35).

9. The sealed collection device (10) of any one of claims 1 to 8, wherein the specimen-retrieval device (130) is a swab.

10. The sealed collection device (10) of claim 9, wherein an upper portion of the specimen-retrieval device (130) is snapped-off before it is contained within the collection device (10).

11. The sealed collection device (10) of any one of claims 1 to 10, wherein the collection device (10) contains a biological specimen.

12. A method for obtaining a fluid substance from the sealed collection device (10) of any one of claims 1 to 10, the method comprising the steps of:
(a) penetrating the cap (20) with a fluid transfer device (70);
(b) entering the collection device (10) so that the specimen-retrieval device (130) does not substantially interfere with the movement of the fluid transfer device (70);
(c) drawing the fluid substance into the fluid transfer device (70); and
(d) removing the fluid transfer device (70) from the collection device (10).

13. The method of claim 12, wherein air is vented from within the collection device (10) as the fluid transfer device (70) enters the collection device (10).

14. The method of claim 12 or 13, wherein the fluid transfer device (70) is an air displacement pipette tip.

15. The method of any one of claims 12 to 14 further comprising performing a nucleic acid-based assay with the fluid substance removed from the collection device (10) in step (d).

## Patentansprüche

1. Eine abgedichtete Sammelvorrichtung (10) mit:
einem Behälter (50) mit einer Seitenwand (58), welche eine Innenoberfläche (59) aufweist,
einer Kappe (20), welche die abgedichtete Sammelvorrichtung abdichtet und eine Innenwand (33) mit einer Außenoberfläche (37) aufweist, welche sich in dem Behälter (50) der Innenoberfläche (59) der Seitenwand (58) des Behälters (50) zugewandt erstreckt; und
einer Proben-Entnahmevorrichtung (130), welche sich im Inneren des Behälters (50) erstreckt, wobei die Kappe (20) entlang eines Fluidtransfer-Weges der Fluidtransfer-Vorrichtung (70) durchdringbar ist und sich der Weg durch die Kappe (20) in den Behälter (50) hinein erstreckt; und wobei sich die Proben-Entnahmevorrichtung (130) entlang der Innenoberfläche (59) der Seitenwand (58) des Behälters (50) erstreckt, **dadurch gekennzeichnet, dass** ein Ende der Proben-Entnahmevorrichtung (130) zwischen der Außenoberfläche (37) der Innenwand (33) der Kappe (20) und der dieser Außenoberfläche zugewandten Innenoberfläche (59) der Seitenwand (58) des Behälters (50) derart eingepasst ist, dass die Proben-Entnahmevorrichtung (130) ausreichend von dem Fluidtransfer-Weg der Fluidtransfer-Vorrichtung (70) isoliert ist.

2. Die abgedichtete Sammelvorrichtung (10) nach Anspruch 1, wobei die Innenwand (33) der Kappe (20) eine im Wesentlichen konische Form aufweist.

3. Die abgedichtete Sammelvorrichtung (10) nach Anspruch 1 oder 2, weiter versehen mit einer durchdringbaren Dichtung (80), welche eine Öffnung der Kappe (20) oberhalb der Innenwand (33) der Kappe (20) bedeckt.

4. Die abgedichtete Sammelvorrichtung (10) nach Anspruch 3, wobei die Kappe weiter mit einer Packung (90) versehen ist, welches zwischen der Innenwand (33) der Kappe (20) und der Dichtung (80) angeordnet ist, wobei die Packung (90) derart ausgebildet ist, dass diese das Ausströmen eines Aerosols aus der Sammelvorrichtung (10) verhindert.

5. Die abgedichtete Sammelvorrichtung (10) nach Anspruch 3 oder 4, wobei die Dichtung (80) eine Metallfolie aufweist.

6. Die abgedichtete Sammelvorrichtung (10) nach Anspruch 3 oder 4, wobei die Dichtung (80) Kunststoff aufweist.

7. Die abgedichtete Sammelvorrichtung (10) nach einem der Ansprüche 1 bis 6, wobei die Kappe (20) im Gießverfahren verarbeiteten Kunststoff aufweist.

8. Die abgedichtete Sammelvorrichtung (10) nach einem der Ansprüche 1 bis 7, wobei die Innenwand (33) der Kappe (20) aus im Gießverfahren verarbeitetem Kunststoff besteht und eine Mehrzahl von Rillen (35) aufweist.

9. Die abgedichtete Sammelvorrichtung (10) nach einem der Ansprüche 1 bis 8, wobei die Proben-Entnahmevorrichtung (130) ein Tupfer ist.

10. Die abgedichtete Sammelvorrichtung (10) nach Anspruch 9, wobei ein oberer Abschnitt der Proben-Entnahmevorrichtung (130) abgebrochen wird, bevor diese in der Sammelvorrichtung (10) untergebracht wird.

11. Die abgedichtete Sammelvorrichtung (10) nach einem der Ansprüche 1 bis 10, wobei die Sammelvorrichtung (10) eine biologische Probe enthält.

12. Ein Verfahren zum Entnehmen einer flüssigen Substanz aus der abgedichteten Sammelvorrichtung (10) nach einem der Ansprüche 1 bis 10, wobei das Verfahren die folgenden Verfahrensschritte aufweist:
(a) Durchdringen der Kappe (20) mit einer Fluidtransfer-Vorrichtung (70);
(b) Eindringen mit der Sammelvorrichtung (10) derart, dass die Proben-Entnahmevorrichtung (130) die Bewegung der Fluidtransfer-Vorrichtung (70) nicht wesentlich beeinträchtigt;
(c) Einziehen der flüssigen Substanz in die Fluidtransfer-Vorrichtung (70); und
(d) Entfernen der Fluidtransfer-Vorrichtung (70) aus der Sammelvorrichtung (10).

13. Das Verfahren nach Anspruch 12, wobei eine Entgasung aus dem Inneren der Sammelvorrichtung erfolgt, während die Fluidtransfer-Vorrichtung (70) in die Sammelvorrichtung (10) eindringt.

14. Das Verfahren nach Anspruch 12 oder 13, wobei die Fluidtransfer-Vorrichtung (70) eine Luftverdrängungs-Pipettenspitze ist.

15. Das Verfahren nach einem der Ansprüche 12 bis 14, wobei weiter ein Nukleinsäure-Assay mit der flüssigen Substanz durchgeführt wird, welche von der Sammelvorrichtung (10) im Schritt (d) entnommen wurde.

## Revendications

1. Dispositif de collecte fermé de manière étanche (10) comprenant :
un récipient (50) ayant une paroi latérale (58) avec une surface intérieure (59),
un bouchon (20) fermant le dispositif de collecte fermé de manière étanche et ayant une paroi intérieure (33) avec une surface extérieure (37) s'étendant dans le récipient à l'opposé de la surface intérieure (59) de la paroi latérale (58) du récipient (50) ; et
un dispositif de prélèvement de spécimen (130) s'étendant dans le récipient (50),
le bouchon (20) pouvant être percé suivant une voie de transfert de fluide d'un dispositif de transfert de fluide (70), la voie s'étendant à travers le bouchon (20) et dans le récipient (50) ; et le dispositif de prélèvement de spécimen (130) s'étendant le long de la surface intérieure (59) de la paroi latérale (58) du récipient (50) ;
**caractérisé en ce qu'**une extrémité du dispositif de prélèvement de spécimen (130) est agencée entre la surface extérieure (37) de la paroi intérieure (33) du bouchon (20) et la surface intérieure opposée (59) de la paroi latérale (58) du récipient (50) de sorte que le dispositif de prélèvement de spécimen (130) soit suffisamment isolé de la voie du dispositif de transfert de fluide (70).

2. Dispositif de collecte fermé de manière étanche (10) selon la revendication 1, dans lequel la paroi intérieure (33) du bouchon (20) présente une forme généralement conique.

3. Dispositif de collecte fermé de manière étanche (10) selon la revendication 1 ou 2 comprenant en outre un joint d'étanchéité pénétrable (80) recouvrant une ouverture dans le bouchon (20) au-dessus de la paroi intérieure (33) du bouchon (20).

4. Dispositif de collecte fermé de manière étanche (10) selon la revendication 3, le bouchon comprenant en outre une mèche (90) positionnée entre la paroi intérieure (33) du bouchon (20) et le joint d'étanchéité (80), la mèche (90) étant adaptée pour empêcher la libération d'aérosols provenant du dispositif de collecte (10).

5. Dispositif de collecte fermé de manière étanche (10) selon la revendication 3 ou 4, dans lequel le joint d'étanchéité (80) comprend une feuille métallique.

6. Dispositif de collecte fermé de manière étanche (10) selon la revendication 3 ou 4, dans lequel le joint d'étanchéité (80) comprend un plastique.

7. Dispositif de collecte fermé de manière étanche (10) selon l'une quelconque des revendications 1 à 6, dans lequel le bouchon (20) comprend un plastique moulé.

8. Dispositif de collecte fermé de manière étanche (10) selon l'une quelconque des revendications 1 à 7, dans lequel la paroi intérieure (33) du bouchon (20) est un plastique moulé comprenant une pluralité de stries (35).

9. Dispositif de collecte fermé de manière étanche (10) selon l'une quelconque des revendications 1 à 8, dans lequel le dispositif de prélèvement de spécimen (130) est un écouvillon.

10. Dispositif de collecte fermé de manière étanche (10) selon la revendication 9, dans lequel une partie supérieure du dispositif de prélèvement de spécimen (130) est cassée avant qu'il soit contenu dans le dispositif de collecte (10).

11. Dispositif de collecte fermé de manière étanche (10) selon l'une quelconque des revendications 1 à 10, dans lequel le dispositif de collecte (10) contient un spécimen biologique.

12. Procédé pour obtenir une substance fluide provenant du dispositif de collecte fermé de manière étanche (10) selon l'une quelconque des revendications 1 à 10, le procédé comprenant les étapes consistant à :
(a) pénétrer le bouchon (20) avec un dispositif de transfert de fluide (70) ;
(b) entrer dans le dispositif de collecte (10) de sorte que le dispositif de prélèvement de spécimen (130) n'interfère pas sensiblement avec le mouvement du dispositif de transfert de fluide (70) ;
(c) aspirer la substance fluidique dans le dispositif de transfert de fluide (70) ; et
(d) retirer le dispositif de transfert de fluide (70) du dispositif de collecte (10).

13. Procédé selon la revendication 12, dans lequel de l'air est évacué depuis l'intérieur du dispositif de collecte (10) à mesure que le dispositif de transfert de fluide (70) pénètre dans le dispositif de collecte (10).

14. Procédé selon la revendication 12 ou 13, dans lequel le dispositif de transfert de fluide (70) est un embout de pipette à déplacement d'air.

15. Procédé selon l'une quelconque de revendication 12 à 14 consistant en outre à réaliser un essai à base d'acide nucléique avec la substance fluidique extraite du dispositif de collecte (10) dans l'étape (d).
